# FASCICULE DE BREVET EUROPEEN

(11) **EP 2 419 422 B1**
(45) Date de publication et mention de la délivrance du brevet: **15.10.2014**
(21) Numéro de dépôt: 10717194.4
(22) Date de dépôt: 16.04.2010
(51) Int. Cl.: C07D 403/06, C07D 403/12, A61K 31/4166, A61P 35/00

(54) **DÉRIVÉS D'IMIDAZOLIDINE-2,4-DIONE ET LEUR UTILISATION COMME MÉDICAMENT**
IMIDAZOLIDIN-2,4-DIONDERIVATE UND DEREN VERWENDUNG ALS MEDIKAMENT
IMIDAZOLIDINE-2,4-DIONE DERIVATIVES AND USE THEREOF AS A MEDICAMENT

(30) Priorité: 17.04.2009 FR 0901864
(43) Date de publication de la demande: 22.02.2012
(73) Titulaire: IPSEN PHARMA S.A.S., 92100 Boulogne-Billancourt (FR)
(72) Inventeur: BIGG, Dennis, F-91190 Gif-sur-Yvette (FR); AUVIN, Serge, F-91120 Palaiseau (FR); LANCO, Christophe, F-91410 Dourdan (FR); PREVOST, Grégoire, F-92160 Antony (FR)
(74) Mandataire: Audonnet, Nathalie
(86) Numéro de dépôt international: PCT/FR2010/000316
(87) Numéro de publication internationale: WO 2010/119194

(56) Documents cités:
- WO-A-2008/017381
- US-A- 3 684 774
- WENQING GAO ET AL: "Pharmacokinetics and Pharmacodynamics of Nonsteroidal Androgen Receptor Ligands" PHARMACEUTICAL RESEARCH, KLUWER ACADEMIC PUBLISHERS-PLENUM PUBLISHERS, NE, vol. 23, no. 8, 14 juillet 2006 (2006-07-14), pages 1641-1658, XP019405174 ISSN: 1573-904X
- DYER E ET AL: "Preparation of polyhydrouracils and polyiminoimidazolidinones" JOURNAL OF POLYMER SCIENCE - PART A - POLYMER CHEMISTRY, WILEY & SONS, HOBOKEN, NJ, US, vol. 7, no. 3, 1 janvier 1969 (1969-01-01) , pages 833-849, XP002311320 ISSN: 0887-624X
- FENG S ET AL: "Bis-huperzine B: Highly potent and selective acetylcholinesterase inhibitors" JOURNAL OF MEDICINAL CHEMISTRY 20050210 US, vol. 48, no. 3, 10 février 2005 (2005-02-10), pages 655-657, XP002554420 ISSN: 0022-2623
- KAGABU S ET AL: "SYNTHESIS OF ALKYLENE-TETHERED BIS-IMIDACLOPRID DERIVATIVES AS HIGHLY INSECTICIDAL AND NERVE-EXCITING AGENTS WITH POTENT AFFINITY TO Ä3HÜ IMIDACLOPRID-BINDING SITES ON NICOTINIC ACETYLCHOLINE RECEPTOR" JOURNAL OF PESTICIDE SCIENCE - NIPPON NOYAKU GAKKAISHI, NIPPON NOYAKU GAKKAI, TOKYO, JP, vol. 27, no. 3, 1 janvier 2002 (2002-01-01), pages 249-256, XP008023482 ISSN: 0385-1559

## Description

### DOMAINE DE L'INVENTION

La présente demande a pour objet de nouveaux dérivés d'imidazolidine-2,4-dione. Ces produits ont une activité anti-proliférative. Ils sont particulièrement intéressants pour traiter les états pathologiques et les maladies liés à une prolifération cellulaire anormale tels que les cancers. L'invention concerne également des compositions pharmaceutiques contenant lesdits produits et leur utilisation pour la préparation d'un médicament.

### ETAT DE LA TECHNIQUE

De nos jours, le cancer constitue encore une des causes majeures de décès et ce malgré de nombreuses molécules sur le marché des médicaments.

II est donc nécessaire d'identifier de nouvelles molécules plus puissantes permettant une meilleure réponse antitumorale et ce par une bonne activité inhibitrice de la prolifération de colonies cellulaires tumorales.

De telles molécules sont donc particulièrement intéressantes pour traiter les états pathologiques liés à une prolifération cellulaire anormale. Elles peuvent ainsi être utilisées pour le traitement de tumeurs ou de cancers, par exemple ceux de l'oesophage, de l'estomac, des intestins, du rectum, de la cavité orale, du pharynx, du larynx, du poumon, du colon, du sein, du cervix uteri, du corpus endométrium, des ovaires, de la prostate, des testicules, de la vessie, des reins, du foie, du pancréas, des os, des tissus conjonctifs, de la peau tels que les mélanomes, des yeux, du cerveau et du système nerveux central, ainsi que le cancer de la thyroïde, la leucémie, la maladie de Hodgkin, les lymphomes autres que ceux de Hodgkin, les mélanomes multiples et autres cancers.

Il est d'intérêt particulier de trouver des thérapies aux cancers hormonodépendants, aux tumeurs exprimant des récepteurs aux androgènes, et aux cancers du sein et de la prostate.

L'utilisation des anti-androgènes dans le cancer de la prostate est basée sur leur propriété à entrer en compétition avec les agonistes naturels du récepteur des androgènes. Cependant l'efficacité de ces anti-androgènes semble limitée dans le temps, les patients finissant par échapper aux traitements. Plusieurs hypothèses à cet échappement ont été développées montrant une activité agoniste en lieu et place d'une activité antagoniste de ces molécules (Veldscholte J, Berrevoets CA, Brinkmann AO, Grootegoed JA, Mulder E.Biochemistry 1992 Mar 3;31(8):2393-9). Par exemple, le nilutamide est capable de stimuler la croissance de cellules humaines de cancer de prostate en culture. En complément de ces indications expérimentales, des données cliniques supportent aussi ce rôle délétère des anti-androgènes (Akimoto S. ;Antiandrogen withdrawal syndrome Nippon Rinsho. 1998 Aug;56(8):2135-9. Paul R, Breul J. Antiandrogen withdrawal syndrome associated with prostate cancer therapies: incidence and clinical significance Drug Saf. 2000 Nov;23(5):381-90).

Ici la demanderesse a identifié des composés montrant une activité anti-proliférative de la tumeur prostatique qui de manière surprenante ne montrent pas d'activité agoniste à des concentrations où le nilutamide se comporte comme un agoniste. Cette différence de comportement sur la prolifération des nouveaux composés par rapport au nilutamide est supportée par leur capacité à induire la disparition des récepteurs androgènes sous leur forme protéique. Le nilutamide n'a lui aucun effet sur ce taux de récepteur.

Les propriétés de ces nouvelles molécules doivent permettre une meilleure prise en charge du cancer de la prostate évitant l'échappement aux anti-androgènes actuels.

De plus, les composés de la présente invention peuvent également être utilisés pour traiter les pathologies liées à la présence de récepteurs aux androgènes comme par exemple l'hyperplasie bénigne de la prostate, la prostamégalie, l'acné, l'alopécie androgénique, l'hirsutisme etc...

### RESUME DE L'INVENTION

L'invention a donc pour objet des composés de formule générale **(I)** 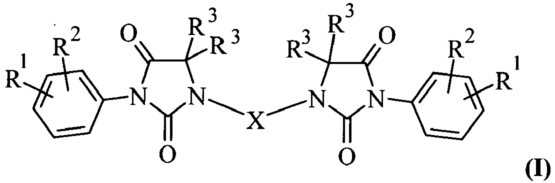 dans laquelle :
R¹ représente un radical cyano, nitro, amino, -NHCOOR⁴ ou -NHCOR⁴ ;
R² représente un radical halo, alkyle, haloalkyle, ou alkoxy ;
R³ représente un radical alkyle ou un atome d'hydrogène ; ou bien les deux radicaux R³ forment ensemble avec l'atome de carbone auquel ils se rattachent un cycloalkyle comportant de 3 à 4 chaînons ;
X représente
   - soit une chaîne alkylène de 3 à 7 atomes de carbone, linéaire ou ramifiée, cette chaîne pouvant contenir un ou plusieurs chaînons identiques ou différents supplémentaires choisis parmi -O-, -N(R⁵)-, -S-, -SO- ou -SO₂- ; ou
   - soit un groupe 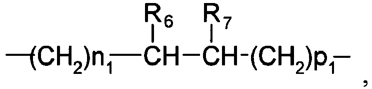 où n1 et p1 sont deux entiers dont la somme ni + p1 est un nombre entier choisi parmi 2, 3, 4 ou 5 ;
      R⁶ et R⁷ forment ensemble une liaison covalente, ou R⁶ et R⁷ forment ensemble avec les atomes de carbone auxquels ils sont reliés le cycle 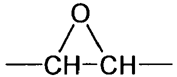 ou un cycloalkyle comportant de 3 à 6 chaînons ;
      R⁴ représente un radical alkyle, aryle, ou hétéroaryle ;
      R⁵ représente un hydrogène, un radical alkyle, ou aralkyle ;
ou un sel pharmaceutiquement acceptable de ce dernier.

De préférence, X représente une chaîne alkylène de 3 à 7 atomes de carbone, linéaire ou ramifiée, cette chaîne pouvant contenir un ou plusieurs chaînons identiques ou différents supplémentaires choisis parmi -O-, -N(R⁵)-, -S-, -SO- ou -SO₂-.

De façon plus préférentielle, X représente une chaîne alkylène pouvant contenir un seul chaînon choisi parmi -O-, -N(R⁵)-, -S-, -SO- ou -SO₂-.

Selon une variante, X représente un groupe 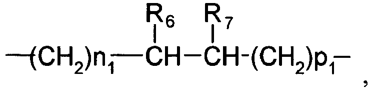 où n1 et p1 sont deux entiers dont la somme n1 + p1 est un nombre entier choisi parmi 2, 3, 4 et 5 ;
R⁶ et R⁷ forment ensemble une liaison covalente, ou R⁶ et R⁷ forment ensemble avec les atomes de carbone auquels ils sont reliés le cycle 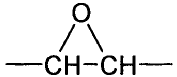 ou un cycloalkyle comportant de 3 à 6 chaînons ;

De préférence, n1 et p1 sont égaux.

De préférence, la somme ni + p1 est égale à 2. De préférence, la somme n1 + p1 est égale à 3. De préférence, la somme n1 + p1 est égale à 4. De préférence, la somme n1 + p1 est égale à 5.

Selon une autre variante, X représente un groupe -(CH₂)n₂-X'-(CH₂)p₂, et X' représente un groupement -O-, -N(R⁵)- ou -S-, -SO-, -SO₂-, -CH₂- ou 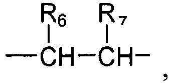 et n2 et p2 sont des entiers dont la somme n2 + p2 est soit un nombre entier choisi parmi 3, 4, 5, 6 et 7 lorsque X' représente un groupement -O-, -N(R⁵)-, -S-, -SO-, -SO₂- ou soit un nombre entier choisi parmi 2, 3, 4 et 5 lorsque X' représente un groupement -CH₂- ou

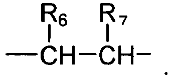

De préférence, X' représente un groupe

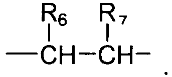

De préférence, X' représente un groupement -O-, -N(R⁵)- ou -(CH₂)-.

De préférence, n2 et p2 sont égaux.

De préférence, la somme n2 + p2 est égale à 2. De préférence, la somme n2 + p2 est égale à 3. De préférence, la somme n2 + p2 est égale à 4. De préférence, la somme n2 + p2 est égale à 5. De préférence, la somme n2 + p2 est égale à 6. De préférence, la somme n2 + p2 est égale à 7.

De préférence, R³ représente un radical alkyle ou les deux radicaux R³ forment ensemble avec l'atome de carbone auquel ils se rattachent un cycloalkyle comportant de 3 à 4 chaînons.

De préférence, R⁴ représente un radical alkyle.

De préférence, R⁵ représente un radical alkyle.

De préférence, X représente une chaîne alkylène de 3 à 7 atomes de carbone linéaire.

De préférence, R¹ est en position para.

De préférence, R² est en position meta.

De préférence, R² représente un radical haloalkyle.

De préférence, R⁶ et R⁷forment ensemble une liaison covalente.

De préférence, R⁶ et R⁷ forment ensemble avec les atomes de carbone auxquels ils sont reliés le cycle

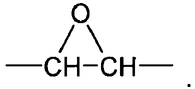

De préférence, R⁶ et R⁷ forment ensemble avec les atomes de carbone auxquels ils sont reliés un cycloalkyle comportant de 3 à 6 chaînons.

Selon une autre variante, R³ représente un radical alkyle ou un hydrogène ; et préférentiellement un radical alkyle.

Préférentiellement, R⁴ représente un radical alkyle. et R⁵ représente un radical alkyle.

De manière préférentielle,
R¹ représente un radical cyano, nitro, amino, -NHCOOR⁴ ou -NHCOR⁴ ;
R² représente un radical halo, alkyle, haloalkyle, alkoxy ;
R³ représente un radical alkyle ;
X représente une chaîne alkylène de 3 à 7 atomes de carbone, linéaire ou ramifiée, cette chaîne pouvant contenir un chaînon -O- ou -N(R⁵)- supplémentaire ;
R⁴ représente un radical alkyle ;
et R⁵ représente un radical alkyle.

Encore plus préférentiellement, R¹ représente un radical cyano, nitro, ou -NHCOOR⁴.

Très préférentiellement également, X représente une chaîne alkylène de 4 à 7 atomes de carbone, cette chaîne pouvant contenir un chaînon -O- supplémentaire.

De préférence, le radical alkyle représente un groupement méthyle.

Le composé de formule (I) est de préférence choisi parmi :
- 1,1'-butane-1,4-diylbis {5,5-dimethyl-3-[4-nitro-3-(trifluoromethyl)-phenyl]imidazolidine-2,4-dione}
- 1,1'-pentane-1,5-diylbis{5,5-dimethyl-3-[4-nitro-3-(trifluoromethyl)-phenyl]imidazolidine-2,4-dione}
- 1,1'-hexane-1,6-diylbis{5,5-dimethyl-3-[4-nitro-3-(trifluoromethyl)-phenyl]imidazolidine-2,4-dione}
- 1,1'-heptane-1,7-diylbis{5,5-dimethyl-3-[4-nitro-3-(trifluoromethyl)-phenyl]imidazolidine-2,4-dione}
- 4,4'-[pentane-1,5-diylbis(4,4-dimethyl-2,5-dioxoimidazolidine-3,1-diyl)] bis[2-(trifluoromethyl)benzonitrile]
- 1,1'-(3-methylpentane-1,5-diyl)bis{5,5-dimethyl-3-[4-nitro-3-(trifluoromethyl)phenyl]imidazolidine-2,4-dione}
- 1,1'-(oxydiethane-2,1-diyl)bis{5,5-dimethyl-3-[4-nitro-3-(trifluoromethyl)-phenyl]imidazolidine-2,4-dione}
- 1,1'-pentane-1,5-diylbis{3-[4-amino-3-(trifluoromethyl)phenyl]-5,5-dimethylimidazolidine-2,4-dione}
- N,N'-(pentane-1,5-diylbis{(4,4-dimethyl-2,5-dioxoimidazolidine-3,1-diyl)[2-(trifluoromethyl)-4,1-phenylene]})diacetamide
- 1,1'-pentane-1,5-diylbis[5,5-dimethyl-3-(3-methyl-4-nitrophenyl)-imidazolidine-2,4-dione]
- 1,1'-pentane-1,5-diylbis{5,5-dimethyl-3-[4-nitro-2-(trifluoromethyl)-phenyl]imidazolidine-2,4-dione}
- 1,1'-pentane-1,5-diylbis[3-(3-chloro-4-nitrophenyl)-5,5-dimethyl-imidazolidine-2,4-dione]
- 1,1'-pentane-1,5-diylbis[3-(3-methoxy-4-nitrophenyl)-5,5-dimethyl-imidazolidine-2,4-dione]
- dimethyl {pentane-l,5-diylbis[(4,4-dimethyl-2,5-dioxoimidazolidine-3,1-diyl)(2-methyl-4,1-phenylene)]}biscarbamate
- 4,4'-[pentane-1,5-diylbis(4,4-dimethyl-2,5-dioxoimidazolidine-3,1-diyl)]bis(2-methylbenzonitrile)
- 4,4'-[pentane-1,5-diylbis(4,4-dimethyl-2,5-dioxoimidazolidine-3,1-diyl)]bis(2-chlorobenzonitrile)
- 1,1'-propane-1,3-diylbis {5,5-diméthyl-3 - [4-nitro-3-(trifluorométhyl)-phényl]imidazolidine-2,4-dione}
- 2-{5,5-diméthyl-3-[4-nitro-3-(trifluorométhyl)phényl]-2,4-dioxoimidazolidin-1-yl}-N-(2-{5,5-diméthyl-3-[4-nitro-3-(trifluorométhyl)-phényl]-2,4-dioxoimidazolidin-1-yl}ethyl)-N-méthylethanamine
- 1,1'-(2Z)-but-2-ene-1,4-diylbis{5,5-dimethyl-3-[4-nitro-3-(trifluoromethyl) phenyl]imidazolidine-2,4-dione}
- 4,4'-[pentane-1,5-diylbis(5,7-dioxo-4,6-diazaspiro[2.4]heptane-4,6-diyl)]bis [2-(trifluoromethyl)benzonitrile]
- 4,4'-[(2Z)-but-2-ene-1,4-diylbis(4,4-dimethyl-2,5-dioxoimidazolidine-3,1-diyl)]bis[2-(trifluoromethyl)benzonitrile]
- 4,4'-{(2*R*,3*S*)-oxirane-2,3-diylbis[methanediyl(4,4-dimethyl-2,5-dioxoimidazolidine-3,1-diyl)]}bis[2-(tritluoromethyl)benzonitrile]
- 4,4'-{(1*R*,2*R*)-cyclopropane-1,2-diylbis[methanediyl(4,4-dimethyl-2,5-dioxoimidazolidine-3,1-diyl)]}bis[2-(tritluoromethyl)benzonitrile]
ou un sel pharmaceutiquement acceptable de ce composé ;

En particulier, le composé de formule (I) est choisi parmi :
- 1,1'-butane-1,4-diylbis{5,5-dimethyl-3-[4-nitro-3-(trifluoromethyl)-phenyl]imidazolidine-2,4-dione}
- 1,1'-pentane-1,5-diylbis{5,5-dimethyl-3-[4-nitro-3-(trifluoromethyl)-phenyl]imidazolidine-2,4-dione}
- 1,1'-hexane-1,6-diylbis{5,5-dimethyl-3-[4-nitro-3-(trifluoromethyl)-phenyl]imidazolidine-2,4-dione}
- 1,1'-heptane-1,7-diylbis{5,5-dimethyl-3-[4-nitro-3-(trifluoromethyl)-phenyl]imidazolidine-2,4-dione}
- 4,4'-[pentane-1,5-diylbis(4,4-dimethyl-2,5-dioxoimidazolidine-3,1-diyl)]bis[2-(trifluoromethyl)benzonitrile]
- 1,1'-(3-methylpentane-1,5-diyl)bis{5,5-dimethyl-3-[4-nitro-3-(trifluoromethyl)phenyl]imidazolidine-2,4-dione}
- 1,1'-(oxydiethane-2,1-diyl)bis{5,5-dimethyl-3-[4-nitro-3-(tritluoromethyl)-phenyl]imidazolidine-2,4-dione}
- 1,1'-pentane-1,5-diylbis{3-[4-amino-3-(trifluoromethyl)phenyl]-5,5-dimethylimidazolidine-2,4-dione}
- N,N'-(pentane-1,5-diylbis{(4,4-dimethyl-2,5-dioxoimidazolidine-3,1-diyl)[2-(trifluoromethyl)-4,1-phenylene]})diacetamide
- 1,1'-pentane-1,5-diylbis[5,5-dimethyl-3-(3-methyl-4-nitrophenyl)-imidazolidine-2,4-dione]
- 1,1'-pentane-l,5-diylbis{5,5-dimethyl-3-[4-nitro-2-(trifluoromethyl)-phenyl]imidazolidine-2,4-dionel}
- 1,1'-pentane-1,5-diylbis[3-(3-chloro-4-nitrophenyl)-5,5-dimethyl-imidazolidine-2,4-dione]
- 1,1'-pentane-1,5-diylbis[3-(3-methoxy-4-nitrophenyl)-5,5-dimethyl-imidazolidine-2,4-dione]
- dimethyl {pentane-1,5-diylbis[(4,4-dimethyl-2,5-dioxoimidazolidine-3,1-diyl)(2-methyl-4,1-phenylene)]}biscarbamate
- 4,4'-[pentane-1,5-diylbis(4,4-dimethyl-2,5-dioxoimidazolidine-3,1-diyl)]bis(2-methylbenzonitrile)
- 4,4'-[pentane-1,5-diylbis(4,4-dimethyl-2,5-dioxoimidazolidine-3,1-diyl)]bis(2-chlorobenzonitrile)
- 1,1'-propane-1,3-diylbis{5,5-diméthyl-3-[4-nitro-3-(trifluorométhyl)-phényl]imidazolidine-2,4-dione}
- 2-{5,5-diméthyl-3 -[4-nitro-3-(trifluorométhyl)phényl]-2,4-dioxoimidazolidin-1-yl}-N-(2-{5,5-diméthyl-3-[4-nitro-3-(trifluorométhyl)-phényl]-2,4-dioxoimidazolidin-1-yl}ethyl)-N-méthylethanamine
- 1,1'-(2Z)-but-2-ene-1,4-diylbis{5,5-dimethyl-3-[4-nitro-3-(trifluoromethyl) phenyl]imidazolidine-2,4-dione}
- 4,4'-[pentane-1,5-diylbis(5,7-dioxo-4,6-diazaspiro[2.4]heptane-4,6-diyl)]bis [2-(trifluoromethyl)benzonitrile]
- 4,4'-[(2Z)-but-2-ene-1,4-diylbis(4,4-dimethyl-2,5-dioxoimidazolidine-3,1-diyl)]bis[2-(trifluoromethyl)benzonitrile]
- 4,4'-{(2*R*,3*S*)-oxirane-2,3-diylbis[methanediyl(4,4-dimethyl-2,5-dioxoimidazolidine-3,1-diyl)] }bis[2-(trifluoromethyl)benzonitrile]
- 4,4'-{(1*R*,2*R*)-cyclopropane-1,2-diylbis[methanediyl(4,4-dimethyl-2,5-dioxoimidazolidine-3,1-diyl)] bis[2-(trifluoromethyl)benzonitrile]
ou un sel pharmaceutiquement acceptable de ce composé ;

De préférence, le composé de formule générale (I) est choisi parmi :
- 1,1'-butane-1,4-diylbis{5,5-dimethyl-3-[4-nitro-3-(trifluoromethyl)-phenyl]imidazolidine-2,4-dione}
- 1,1'-pentane-1,5-diylbis{5,5-dimethyl-3-[4-nitro-3-(trifluoromethyl)-phenyl]imidazolidine-2,4-dione}
- 1,1'-(oxydiethane-2,1-diyl)bis{5,5-dimethyl-3-[4-nitro-3-(tritluoromethyl)-phenyl]imidazolidine-2,4-dione}
- 4,4'-[pentane-1,5-diylbis(4,4-dimethyl-2,5-dioxoimidazolidine-3,1-diyl)]bis(2-methylbenzonitrile)
- 1,1'-(2Z)-but-2-ene-1,4-diylbis{5,5-dimethyl-3-[4-nitro-3-(trifluoromethyl) phenyl]imidazolidine-2,4-dione}
ou un sel pharmaceutiquement acceptable de celui-ci.

Plus particulièrement le composé de formule **(I)** est choisi parmi :
- 1,1'-butane-1,4-diylbis{5,5-dimethyl-3-[4-nitro-3-(trifluoromethyl)-phenyl]imidazolidine-2,4-dione}
- 1,1'-pentane-1,5-diylbis{5,5-dimethyl-3-[4-nitro-3-(trifluoromethyl)-phenyl]imidazolidine-2,4-dione}
- 1,1'-(oxydiethane-2,1-diyl)bis{5,5-dimethyl-3-[4-nitro-3-(trifluoromethyl)-phenyl]imidazolidine-2,4-dione}
- 4,4'-[pentane-1,5-diylbis(4,4-dimethyl-2,5-dioxoimidazolidine-3,1-diyl)]bis(2-methylbenzonitrile).
- 1,1'-(2Z)-but-2-ene-1,4-diylbis{5,5-dimethyl-3-[4-nitro-3-(trifluoromethyl) phenyl]imidazolidine-2,4-dione}
ou un sel pharmaceutiquement acceptable de ce composé.

De préférence, le composé de formule générale **(I)** est le 1,1'-pentane-1,5-diylbis{5,5-dimethyl-3-[4-nitro-3-(trifluoromethyl)-phenyl]imidazolidine-2,4-dione} ou le 1,1'-(2Z)-but-2-ene-1,4-diylbis{5,5-dimethyl-3-[4-nitro-3-(trifluoromethyl) phenyl]imidazolidine-2,4-dione}.

Tout particulièrement, le composé de formule **(I)** est le 1,1'-pentane-1,5-diylbis{5,5-dimethyl-3-[4-nitro-3-(trifluoromethyl)-phenyl]imidazolidine-2,4-dione}.

L'invention a également pour objet un procédé de préparation d'un composé de formule **(I),** comprenant une étape qui consiste à
(i) condenser deux équivalents d'arylhydantoïnes de formule générale **(II)** 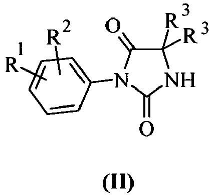 dans laquelle R² et R³ sont tels que définis précédemment et R¹ est un groupement nitro ou cyano, sur un dérivé de formule générale Gp¹-X-Gp², Gp¹ et Gp² étant des groupes partants, et X étant tel que défini précédemment, en présence d'une base forte, pour former le composé de formule générale **(I)** 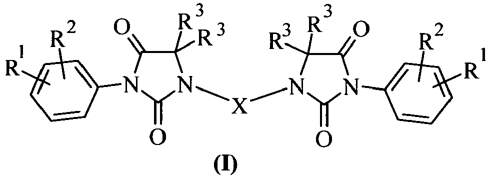 dans laquelle R², R³ et X sont tels que définis précédemment et R¹ est un groupement nitro ou cyano.
   De préférence, la condensation est effectuée par chauffage du mélange réactionnel à une température comprise entre 20 et 100° C, de préférence, entre 45 et 65°C.
   De préférence, la réaction est réalisée dans un solvant polaire aprotique.
   De préférence, le procédé comprend en outre une étape de
(ii) réduction du groupement nitro afin d'obtenir un composé de formule (III) 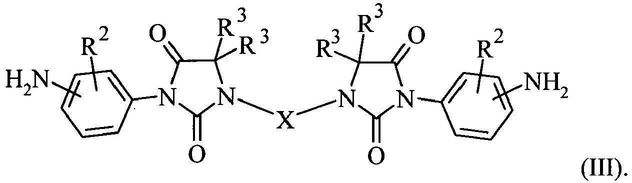
   De préférence, le procédé comprend en outre une étape qui consiste à
(iii) la réaction d'un composé de formule (III) obtenu dans l'étape (ii) avec un chlorure d'acide de formule générale R⁴-COCl dans lequel R⁴ est tel que défini précédemment afin d'obtenir un composé de formule (IV) 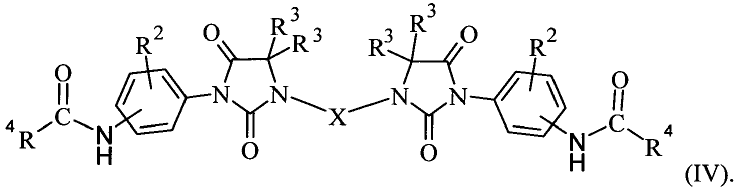
   Selon une variante, le procédé comprend en outre une étape qui consiste à
(iv) la réaction d'un composé obtenu dans l'étape (ii) avec un chloroformiate de formule générale R⁴-O-CO-Cl dans lequel R⁴ est tel que défini précédemment afin d'obtenir un composé de formule (V) 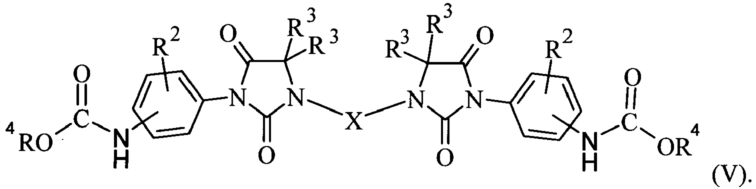
   Selon une autre variante, si R⁶ et R⁷ forment ensemble une liaison covalente, le procédé peut comprendre en outre une étape de :
(v) oxydation du composé de formule (I) dans lequel R⁶ et R⁷ forment ensemble une liaison covalente, de la double liaison ainsi formée par R⁶ et R⁷ afin d'obtenir un composé de formule (VI)

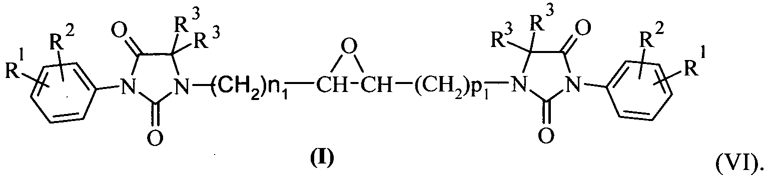

L'invention concerne également en tant que médicament, un composé de formule **(I).**

L'invention concerne également les compositions pharmaceutiques contenant, à titre de principe actif, au moins un composé de formule **(I)** en association avec un support pharmaceutiquement acceptable.

L'invention concerne également l'utilisation d'un composé de formule **(I)**, pour la préparation d'un médicament destiné à traiter les cancers.

De préférence, le médicament est destiné à traiter un cancer hormono-dépendant.

De préférence, le médicament est destiné à traiter un cancer exprimant les récepteurs aux androgènes

De préférence, le médicament est destiné à traiter un cancer du sein ou de la prostate, de préférence, un cancer de la prostate.

### BREVE DESCRIPTION DES FIGURES

La figure 1 représente l'effet des composés des exemples 2 et 19 sur la prolifération cellulaire des LNCaP cultivées en milieu sans stéroïde.
Les figures 2 à 9 représentent les effets des composés des exemples 2, 7, 10, 15, 16, 19, 21, et 22 sur la diminution de l'expression protéique du récepteur aux androgènes.
La figure 10 représente l'effet du nilutamide sur la diminution de l'expression protéique du récepteur aux androgènes.

### DESCRITION DETAILLEE DE MODES DE REALISATION DE L'INTENTION

L'invention a donc pour objet des composés de formule générale **(I)** 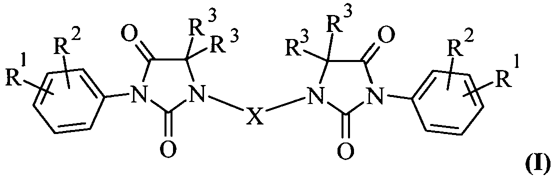 dans laquelle R¹ représente un radical cyano, nitro, amino, -NHCOOR⁴ ou -NHCOR⁴.
R² représente un radical halo, alkyle, haloalkyle, alkoxy. R³ représente un radical alkyle ou un hydrogène. Alternativement, les deux radicaux R³ forment ensemble avec l'atome de carbone auquel ils se rattachent un radical cycloalkyle comportant de 3 à 4 chaînons.
X représente une chaîne alkylène de 3 à 7 atomes de carbone, linéaire ou ramifiée, cette chaîne pouvant contenir un ou plusieurs chaînons identiques ou différents -O-, -N(R⁵)-ou -S-, -SO- ou -SO₂-supplémentaires. Alternativement, X représente un groupe 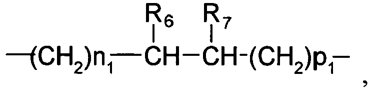 où n1 et p1 sont deux entiers dont la somme n1 + p1 est comprise entre 2 et 5. Par exemple, n1 et p1 valent chacun 1 ou 2, de préférence n1 et p1 valent 1.
R⁶ et R⁷ forment ensemble une liaison covalente. Dans ce cas, X représente alors un groupe (CH2)n1-CH=CH-(CH2)p1.

De façon alternative, R⁶ et R⁷ forment ensemble avec les atomes de carbone auxquels ils sont reliés un cycle 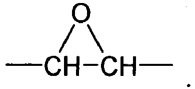 Selon une autre alternative, R⁶ et R⁷ forment ensemble avec les atomes de carbone auxquels ils sont reliés un radical cycloalkyle comportant de 3 à 6 chaînons, par exemple, un cyclobutyle, un cyclopentyle ou un cyclohexyle.
R⁴ représente un radical alkyle, aryle, ou hétéroaryle.
R⁵ représente un hydrogène, un radical alkyle, ou aralkyle.

Les composés de formule (I) peuvent être sous forme d'un sel pharmaceutiquement acceptable.

Par sel pharmaceutiquement acceptable, on entend notamment des sels d'addition d'acides inorganiques tels que chlorhydrate, bromhydrate, sulfate, phosphate, diphosphate et nitrate ou d'acides organiques tels que acétate, maléate, fumarate, tartrate, succinate, citrate, lactate, méthanesulfonate, benzènesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

Dans les définitions indiquées ci-dessus, l'expression halogène (ou halo) représente le radical fluoro, chloro, bromo ou iodo, de préférence chloro, fluoro ou bromo.

Lorsqu'il n'est pas donné plus de précisions, le terme alkyle au sens de la présente invention représente un radical alkyl linéaire ou ramifié comprenant entre 1 et 12 atomes de carbones tels que les radicaux méthyle, éthyle, propyle, isopropyle, butyle, isobutyle, *sec*-butyle et *tert*-butyle, pentyle ou amyle, isopentyle, neopentyle, hexyle ou isohexyle, heptyle, octyle, nonyle, décyle, undécyle ou dodécyle. De manière préférentielle le radical alkyle sera un radical (C₁-C₆)alkyle, c'est-à-dire représentant un radical alkyle ayant de 1 à 6 atomes de carbone tel que défini ci-dessus, ou un radical (C₁-C₄)alkyle représentant un radical alkyle ayant de 1 à 4 atomes de carbone tel que par exemple les radicaux méthyle, éthyle, propyle, isopropyle, butyle, isobutyle, *sec*-butyle et *tert*-butyle. De manière très préférentielle, le radical alkyle est le radical méthyle.

Le terme alkyle dans les expressions alkoxy (ou alkyloxy), ou haloalkyle représente un radical akyle tel que défini ci-dessus.

Plus particulièrement, par haloalkyle, on entend un radical alkyle dont au moins l'un des atomes d'hydrogène (et éventuellement tous) est remplacé par un atome d'halogène (halo) comme par exemple, et préférentiellement trifluorométhyle.

Par cycloalkyle lorsqu'il n'est pas donné plus de précision, on entend un radical cyclique carboné saturé comprenant 3 à 4 chaînons tel que le cyclopropyle ou le cyclobutyle.

Au sens de la présente invention, les radicaux aryles peuvent être de type mono ou polycycliques aromatiques. Les radicaux aryles monocycliques peuvent être choisis parmi les radicaux phényles, tolyles, xylyles, mésityles, cuményles et de préférence phényles. Les radicaux aryles polycycliques peuvent être choisis parmi les radicaux naphtyle, anthryle, phénanthryle, fluorényle. Ils peuvent être optionnellement substitués par un ou plusieurs radicaux identiques ou différents tels que alkyle, haloalkyle, alkoxy, alkoxycarbonyle, alkylcarbonyloxy, halo, cyano, nitro, aryle, aryloxy, aryloxycarbonyl, ou arylcarbonyloxy.

Au sens de la présente invention, le terme aralkyle représente un radical alkyle tel que défini précédemment substitué par un radical aryle tel que défini ci-dessus.

Au sens de la présente invention, le terme hétéroaryle désigne un cycle insaturé aromatique comprenant un ou plusieurs hétéroatomes identiques ou différents choisis parmi N, O et S tels que furyle, thiènyle isoxazolyle, benzothiadiazolyle, pyridinyle, oxazolyle, pyrazolyle, pyrimidinyle ou quinoxalyle.

L'invention a également pour objet un procédé de préparation d'un composé de formule **(I),** comprenant une étape qui consiste à
(i) condenser deux équivalents d'arylhydantoïnes de formule générale **(II)** 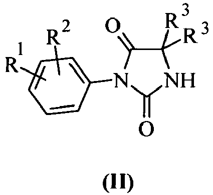 dans laquelle R² et R³ sont tels que définis précédemment et R¹ est un groupement nitro ou cyano, sur un dérivé de formule générale Gp¹-X-Gp², Gp¹ et Gp² étant des groupes partants, et X étant tel que défini précédemment, en présence d'une base forte, pour former le composé de formule générale **(I)** 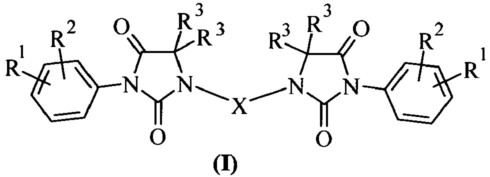 dans laquelle R², R³ et X sont tels que définis précédemment et R¹ est un groupement nitro ou cyano.

### A) Préparation des composés dans lesquels R¹ est un groupement nitro ou cyano :

Les composés de formule générale **(I)** selon l'invention peuvent être préparés selon la voie de synthèse représentée dans le Schéma 1, ci-dessous. Les composés de formule générale **(I)** dans laquelle R¹, R², R³ et X sont tels que décrits ci-dessus peuvent être obtenus en une étape par la condensation de deux équivalents d'arylhydantoïnes intermédiaires de formule générale **(II)** sur un dérivé de formule générale Gp¹-X-Gp² Gp¹ et Gp² étant considérés comme groupes partants, et X étant tel que défini précédemment, tels que par exemple un atome d'halogène ou bien un groupe sulfonate. Cette condensation s'effectue en présence d'une base, telle que par exemple NaH. De préférence, la condensation est effectuée par chauffage du mélange réactionnel à une température comprise entre 20 et 100° C, de préférence entre 45 et 65°C. De préférence, la condensation est effectuée dans un solvant polaire, de préférence dans un solvant polaire aprotique tels que par exemple le THF, le DMF ou le DMSO. Généralement, la réaction est effectuée pendant une durée variant de 1 à 15 heures.

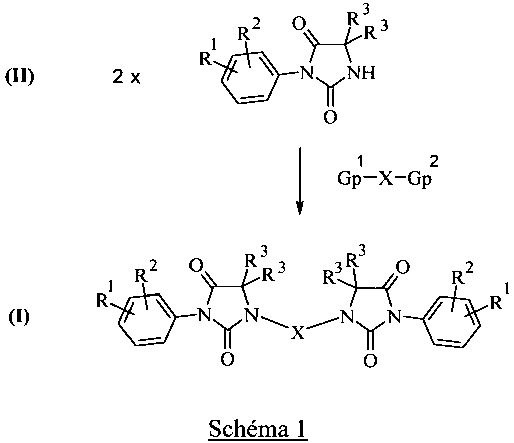

### A.1) Préparation des composés dans lesquels R¹ est un groupement amino

Dans le cas particulier où R¹ est un groupe nitro, R², R³ et X étant tels que décrits ci-dessus, la préparation des dérivés aniline de formule générale **(I)_{A1}** est représentée dans le schéma A1. La réduction des groupements nitro est effectuée par SnCl₂, 2H₂O (J. Heterocyclic Chem. 1987, 24, 927-930 ; Tetrahedron Letters 1984, 25 (8), 839-842) dans un solvant approprié tel que l'acétate d'éthyle.

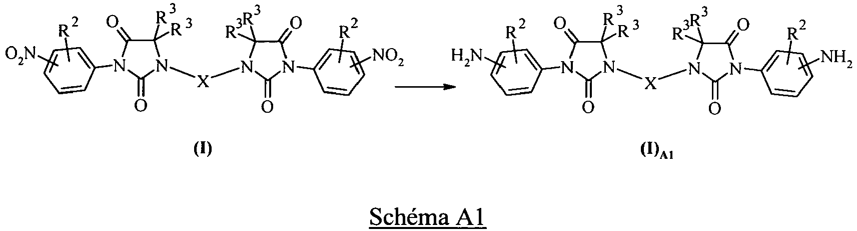

### A.2) Préparation des composés dans lesquels R¹ est un groupement acétamide :

Les dérivés acétamides de formule générale (**I**)**_{A2}** dans lesquels R², R³, R⁴ et X sont tels que décrits ci-dessus, sont accessibles en une étape à partir des dérivés anilines de formule générale (**I**)**_{A1}**, schéma A2. La réaction d'acylation peut être effectuée à l'aide d'un large excès de chlorure d'acide de formule générale R⁴-COCl, par exemple le chlorure d'acétyle, ou bien d'un anhydride de type (R⁴-CO)₂O, tel que l'anhydride acétique, et en utilisant ce réactif en excès comme solvant.

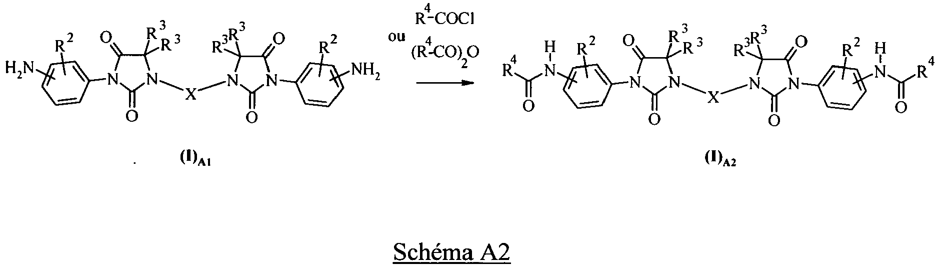

### A.3) Préparation des composés dans lesquels R¹ est un groupement carbamate :

Les dérivés carbamates de formule générale **(I)_{A3}** dans lesquels R², R³, R⁴ et X sont tels que décrits ci-dessus, sont préparés en une étape à partir des dérivés anilines de formule générale **(I)_{A1}**, schéma A3. La formation du carbamate s'effectue à l'aide d'un large excès d'un chloroformiate de formule générale R⁴-O-CO-Cl, en présence d'un solvant anhydre, de préférence un solvant aprotique polaire anhydre. De préférence, on utilise la pyridine anhydre. La réaction est conduite généralement par chauffage à une température comprise entre 60 et 100° C et pour une durée de 12 à 24 heures.

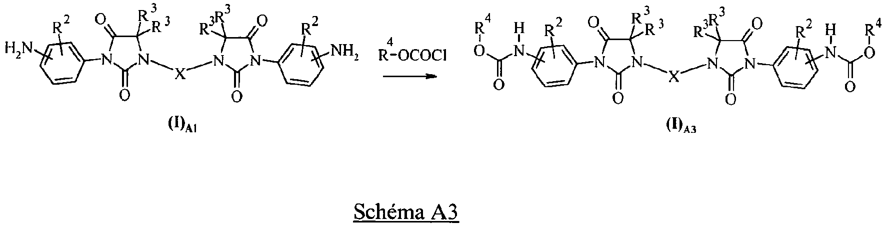

### A.4) Préparation des composés dans lesquels X contient un oxirane.

Dans le cas particulier où X contient une double liaison, R¹, R² et R³ étant tels que décrit ci-dessus, les composés de formules générales **(I)_{A4}** peuvent être obtenus par oxydation des composés de formule générale **(I)** par un agent d'oxydation approprié tel que par exemple l'acide perbenzoique ou l'acide peracétique dans un solvant aprotique La réaction est conduite généralement à température ambiante et pour une durée de 1 à 4 jours.

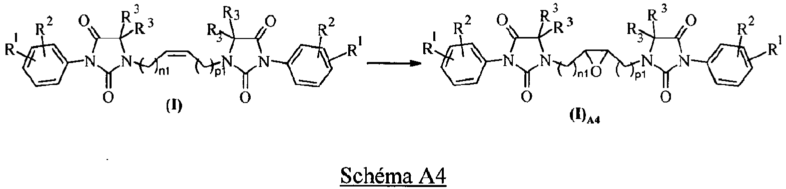

### B) Préparation des intermédiaires de formule générale (II) :

La synthèse des arylhydantoïnes intermédiaires de formule générale **(II)** dans lesquels R¹, R² et R³ sont tels que décrits ci-dessus peuvent être effectuées selon les stratégies décrites dans les schémas ci-dessous :

### B.1) Préparation des arylhydantoïnes par condensation :

La synthèse de l'arylhydantoïne de formule générale **(II),** schéma B1, peut être effectuée par substitution nucléophile de l'atome de fluor porté par le noyau aromatique du composé de formule générale **(II)₁** par l'anion de l'hydantoine de formule générale **(II)₂** généré en présence d'une base telle que, par exemple K₂CO₃. La réaction s'effectue par chauffage à une température comprise entre 65 et 140° C et dans un solvant polaire aprotique tel que, par exemple, le DMF ou le DMSO. La température et le temps de réaction sont fonction du caractère nucléofuge de l'atome de fluor qui est fortement dépendant de la nature de R¹ et R². Les hydantoïnes de formule générale **(II)₂** non commerciales peuvent être préparées selon des méthodes décrites dans la littérature (p. ex. J. Med. Chem. 1984, 27 (12), 1663-8).

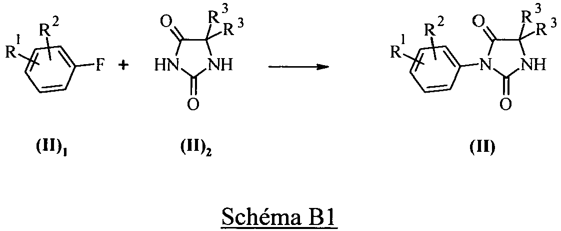

Dans les cas où R¹et R² ne sont pas suffisamment électro-attracteurs pour favoriser la substitution nucléophile aromatique décrite dans le schéma B1, une approche par couplage entre un acide aryl boronique et l'hydantoine de formule générale **(II)₂** en présence d'acétate de cuivre peut être envisagée (Synlett 2006, 14, 2290-2) pour obtenir les composés de formule générale **(II).**

### B.2) Préparation des arylhydantoïnes par construction du cycle hydantoïne à partir d'arylisocyanate :

L'accès aux hydantoines de formule générale **(II)** se fait, dans ce cas, selon un protocole décrit dans Bioorg. Med. Chem. Lett. 2004, 14, 5285.

### B.3) Préparation des arylhydantoïnes par cyclisation à partir des arylurées

La synthèse des arylhydantoïnes intermédiaires de formule générale (II), schéma B3, dans lesquels R¹, R² et R³ sont tels que décrits ci-dessus peut être effectuée par cyclisation d'un intermédiaire de formule générale (II)₃ préparé selon des méthodes décrites dans la littérature (p. ex. Organic Process Research & Development 2002, 6, 759-761). La réaction de cyclisation peut être effectuée par formation intermédiaire de l'halogénure d'acyle puis par chauffage. L'halogénure d'acyle pouvant être généré par un agent d'halogénation tel que par exemple le chlorure d'oxalyle ou le chlorure de thionyle dans un solvant aprotique tel que par exemple le 1-4, dioxane ou le tétrahydrofurane.

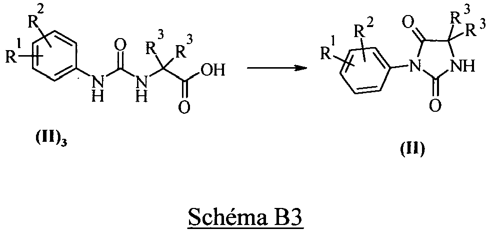

### B.4) Préparation des arylhydantoïnes par construction du cycle hydantoïne à partir des isocyanates d'aminoesters :

Alternativement, les arylhydantoïnes de formule générale **(II)** peuvent être synthétisées à partir d'isocyanate d'aminoesters tel que décrit dans Eur. J Med. Chem. 1984, 19 (3), 261.

La salification des composés de formule (I) peut être effectuée par toute méthode connue de l'homme du métier. Par exemple, la salification peut être réalisée par ajout de base ou d'acide, par exemple, d'hydroxyde de sodium, de potassium, ou d'acide chlorhydrique.

Les composés de formule **(I)** selon la présente invention possèdent d'intéressantes propriétés pharmacologiques. En effet, on a découvert que les composés de formule **(I)** de la présente invention possèdent une activité anti-tumorale (anti-cancéreuse), et plus particulièrement une activité inhibitrice de la prolifération cellulaire des cellules exprimant des récepteurs aux androgènes telles que les cellules prostatiques de type LnCAP. Ainsi, les composés de la présente invention peuvent être utilisés dans différentes applications thérapeutiques. Ils peuvent avantageusement être utilisés pour le traitement des cancers, particulièrement des cancers hormonodépendants, des cancers exprimant des récepteurs aux androgènes et plus particulièrement des cancers du sein et de la prostate. On trouvera ci-après, dans la partie expérimentale, une illustration des propriétés pharmacologiques des composés de l'invention.

La présente demande a donc également pour objet, les composé de formule **(I)** tels que définis précédemment en tant que médicaments.

Également, la présente demande a pour objet les composé de formule **(I)** tels que définis précédemment en tant que médicaments destinés à traiter les maladies prolifératives, préférentiellement les cancers, très préférentiellement les cancers hormonodépendants ou bien les cancers exprimant des récepteurs aux androgènes, ou encore les cancers de la prostate et du sein et de manière très préférentielle les cancers de la prostate.

La présente demande a également pour objet, des compositions pharmaceutiques contenant, à titre de principe actif, au moins un composé de formule **(I)** tel que défini ci-dessus, en association avec un support pharmaceutiquement acceptable.

La présente demande a également pour objet l'utilisation d'un composé de formule **(I)** selon la présente invention, pour la préparation d'un médicament anti-tumoral.

La présente demande a également pour objet l'utilisation d'un composé de formule **(I)** selon la présente invention, pour la préparation d'un médicament destiné à inhiber la prolifération cellulaire.

La présente demande a également pour objet l'utilisation d'un composé de formule **(I)** selon la présente invention, pour la préparation d'un médicament destiné à traiter les maladies prolifératives, préférentiellement les cancers, très préférentiellement les cancers hormono-dépendants ou les cancers exprimant des récepteurs aux androgènes, ou les cancers de la prostate et du sein et de manière très préférentielle les cancers de la prostate.

La composition pharmaceutique peut être sous forme d'un solide, par exemple, des poudres, des granules, des comprimés, des gélules. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrées par voie intraveineuse.

Tous les termes techniques et scientifiques utilisés dans le présent texte ont la signification connue de l'homme de l'art. Par ailleurs, tous les brevets (ou demandes de brevet) ainsi que les autres références bibliographiques sont incorporés par référence.

### Partie expérimentale

Suivant les définitions des groupes variables R¹, R², R³, et X, les composés selon l'invention peuvent être préparés selon les différentes procédures décrites ci-dessus.

Les analyses RMN des exemples 1 à 23 ont été réalisées sur un spectromètre Bruker-Avance II de 400 MHz.

Les exemples sont présentés pour illustrer les procédures ci-dessus et ne doivent en aucun cas être considérés comme une limite à la portée de l'invention.

La terminologie utilisée pour la nomenclature des composés ci-dessous et des exemples est la terminologie IUPAC.

### Exemple 1 : 1,1'-butane-1,4-diylbis{5,5-dimethyl-3-[4-nitro-3-(trifluoromethyl)-phenyl]imidazolidine-2,4-dione}

Sous argon, on ajoute NaH (à 60 %) (22 mg, 0,55 mmole) à une solution de 5,5-dimethyl-3-[4-nitro-3-(trifluoromethyl)phenyl]-imidazolidine-2,4-dione (158 mg, 0,5 mmole) dans le DMF anhydre (8 ml). Un dégagement gazeux accompagne le changement de coloration du milieu réactionnel qui devient orange. L'agitation est maintenue 30 minutes à 23° C avant d'ajouter le 1,4-dibromobutane (30 µl, 0,25 mmole). Le mélange réactionnel est chauffé à 55° C pendant 1 heure avant d'être versé dans une solution aqueuse saturée de NH₄Cl (25 ml) et extrait par AcOEt (2 x 25 ml). Les phases organiques sont rassemblées et lavées successivement par de l'eau (25 ml) et de la saumure (25 ml). Après séchage sur Na₂SO₄, la solution organique est filtrée et concentrée sous vide. Le résidu d'évaporation est purifié sur une colonne de silice (éluant : Heptane/AcOEt : 4/6 jusqu'à 1/9).

Le composé attendu est obtenu sous forme d'un solide couleur jaune clair avec un rendement de 45 %. Point de fusion : 211-212° C.

¹H NMR 400MHz (DMSO-*d*₆) δ 8,32 (d, 2H, Ph) ; 8,21 (d, 2H, Ph) ; 8,08 (dd, 2H, Ph) ; 3,39 (m, 4H, 2 x NCH₂) ; 1,70 (m, 4H, 2 x CH₂) ; 1,49 (s, 12H, 4 x CH₃).

### Exemple 2 : 1,1'-pentane-1,5-diylbis{5,5-dimethyl-3-[4-nitro-3-(trifluoromethyl)-phenyl]imidazolidine-2,4-dione}

Le protocole expérimental utilisé est le même que celui décrit pour la synthèse du composé de l'Exemple 1, le 1,5-diiodopentane remplaçant le 1,4-dibromobutane. Le composé attendu est obtenu sous forme d'un solide blanc avec un rendement de 40 %. Point de fusion : 163-164° C.

¹H NMR 400MHz (DMSO-*d*₆) δ : 8,33 (d, 2H, Ph) ; 8,21 (d, 2H, Ph) ; 8,07 (d, 2H, Ph) ; 3,34 (m, 4H, 2 x NCH₂) ; 1,69 (m, 4H, 2 x CH₂) ; 1,50 (s, 12H, 4 x CH₃) ; 1,41 (m, 2H, CH₂).

### Exemple 3 : 1,1'-hexane-1,6-diylbis{5,5-dimethyl-3-[4-nitro-3-(trifluoromethyl)-phenyl]imidazolidine-2,4-dione}

Le protocole expérimental utilisé est le même que celui décrit pour la synthèse du composé de l'Exemple 1, le 1,6-diiodohexane remplaçant le 1,4-dibromobutane. Le composé attendu est obtenu sous forme d'un solide jaune clair avec un rendement de 27 %. Point de fusion : 187-188° C.

¹H NMR 400MHz (DMSO-*d*₆) δ : 8,31 (d large, 2H, Ph) ; 8,20 (s large, 2H, Ph) ; 8,07 (d large, 2H, Ph) ; 3,32 (m, 4H, 2 x NCH₂) ; 1,64 (m, 4H, 2 x CH₂) ; 1,46 (s, 12H, 4 x CH₃) ; 1,38 (m, 4H, 2 x CH₂).

### Exemple 4 : 1,1'-heptane-1,7-diylbis{5,5-dimethyl-3-[4-nitro-3-(trifluoromethyl)-phenyl]imidazolidine-2,4-dione}

Le protocole expérimental utilisé est le même que celui décrit pour la synthèse du composé de l'Exemple 1, le 1,7-dibromoheptane remplaçant le 1,4-dibromobutane. Le composé attendu est obtenu sous forme d'un solide jaune clair avec un rendement de 35 %. Point de fusion : 137-138° C.

¹H NMR 400MHz (DMSO-*d*₆) δ : 8,31 (d, 2H, Ph) ; 8,19 (d, 2H, Ph) ; 8,06 (dd, 2H, Ph) ; 3,30 (m, 4H, 2 x NCH₂) ; 1,64 (m, 4H, 2 x CH₂) ; 1,46 (s, 12H, 4 x CH₃) ; 1,36 (m, 6H, 3x CH₂).

### Exemple 5 : 4,4'-[pentane-1,5-diylbis(4,4-dimethyl-2,5-dioxoimidazolidine-3,1-diyl)]bis[2-(trifluoromethyl)benzonitrile]

### 5.1) 4-(4,4-dimethyl-2,5-dioxoimidazolidin-1-yl)-2-(trifluoromethyl)benzonitrile

Un mélange de 4-fluoro-2-(trifluorométhyl)benzonitrile (5,67 g, 30 mmoles), 5,5-diméthyl-hydantoine (7,68 g, 60 mmoles), K₂CO₃ (8,28 g, 60 mmoles) dans le DMF (45 ml) est réparti en parts égales dans trois tubes destinés au four à micro-onde. Sous agitation magnétique, chaque tube est irradié à 140° C pendant 20 minutes. Les masses réactionnelles sont ensuite réunies, versées dans de l'eau (200 ml) et extraites par AcOEt (2 x 75 ml). Les phases organiques sont réunies, lavées par de la saumure, séchées sur Na₂SO₄ et filtrées. Le filtrat est concentré sous pression réduite et le résidu cristallisé dans Et₂O (25 ml). Après recristallisation dans EtOH (75 ml), la poudre est filtrée et séchée sous vide. Le composé attendu est obtenu sous forme d'un solide blanc avec un rendement de 46 % (4,1 g). Point de fusion : 212-213° C.

¹H NMR 400MHz (DMSO-*d*₆) δ : 8,80 (s, 1H, NH) ; 8,29 (d, 1H, Ph) ; 8,18 (s, 1H, Ph) ; 8,02 (d, 1H, Ph) ; 1,42 (s, 6H, 2 x CH₃).

### 5.2) 4,4'-[pentane-1,5-diylbis(4,4-dimethyl-2,5-dioxoimidazolidine-3,1-diyl)] bis [2-(trifluoromethyl)benzonitrile]

Le protocole expérimental utilisé est le même que celui décrit pour la synthèse du composé de l'Exemple 2, l'intermédiaire 5.1 remplaçant le 5,5-dimethyl-3-[4-nitro-3-(trifluoromethyl)-phenyl]-imidazolidine-2,4-dione. Le composé attendu est obtenu sous forme d'un solide blanc avec un rendement de 50 % (330 mg). Point de fusion : 167-169° C.

¹H NMR 400MHz (DMSO-*d*₆) δ : 8,28 (d, 2H, Ph) ; 8,18 (s, 2H, Ph) ; 8,02 (d, 2H, Ph) ; 3,30 (m, 4H, 2 x NCH₂) ; 1,67 (m, 4H, 2 x CH₂) ; 1,46 (s, 12H, 4 x CH₃) ; 1,40 (m, 2H, CH₂).

### Exemple 6 : 1,1'-(3-methylpentane-1,5-diyl)bis{5,5-dimethyl-3-[4-nitro-3-(trifluoromethyl)phenyl]imidazolidine-2,4-dione}

Le protocole expérimental utilisé est le même que celui décrit pour la synthèse du composé de l'Exemple 1, le 1,5-dibromo-3-méthylpentane remplaçant le 1,4-dibromobutane. Le composé attendu est obtenu sous forme d'une mousse couleur jaune clair avec un rendement de 39 %.

¹H NMR 400MHz (DMSO-*d*₆) δ : 8,30 (d, 2H, Ph) ; 8,20 (d, 2H, Ph) ; 8,07 (dd, 2H, Ph) ; 3,38 (m, 4H, 2 x NCH₂) ; 1,72 (m, 4H, 2 x CH₂) ; 1,53 (m, 1H, CH) ; 1,49 (s, 12H, 4 x CH₃) ; 1,00 (d, 3H, CH₃).

### Exemple 7: 1,1'-(oxydiethane-2,1-diyl)bis{5,5-dimethyl-3-[4-nitro-3-(trifluoromethyl)-phenyl]imidazolidine-2,4-dione}

Le protocole expérimental utilisé est le même que celui décrit pour la synthèse du composé de l'Exemple 1, le bis-(2-bromoethyl)ether remplaçant le 1,4-dibromobutane Le composé attendu est obtenu sous forme d'un solide blanc avec un rendement de 70 %. Point de fusion : 186-188° C.

¹H NMR 400MHz (DMSO-*d*₆) δ : 8,28 (d, 2H, Ph) ; 8,16 (d, 2H, Ph) ; 8,03 (dd, 2H, Ph) ; 3,67 (t, 4H, 2 x CH₂) ; 3,52 (t, 4H, 2 x CH₂) ; 1,47 (s, 12H, 4 x CH₃).

### Exemple 8: 1,1'-pentane-1,5-diy{bis{3-[4-amino-3-(trifluoromethyl)phenyl]-5,5-dimethylimidazolidine-2,4-dione}

Un mélange du composé de l'Exemple 2 (410 mg, 0,58 mmole) et de SnCl₂, 2H₂O (1,32 g, 5,8 mmoles) dans AcOEt (10 ml) est chauffé à 80° C pendant 90 minutes. Le milieu réactionnel est ensuite refroidi à 0° C avant d'être versé dans une solution aqueuse saturée de Na₂CO₃ (40 ml). Le mélange hétérogène ainsi obtenu est filtré sur célite et rincé par AcOEt (2 x 50 ml). Après décantation, les phases organiques sont réunies, séchées sur Na₂SO₄, filtrées et le solvant évaporé sous pression réduite. Le résidu obtenu est repris par un mélange heptane/AcOEt pour conduire à un solide blanc, après filtration, avec un rendement de 78 % (290 mg). Point de fusion : 108-109° C.

¹H NMR 400MHz (DMSO-*d*₆) δ : 7,32 (d, 2H, Ph) ; 7,23 (dd, 2H, Ph) ; 6,85 (d, 2H, Ph) ; 5,80 (s, 4H, 2 x NH₂) ; 3,27 (m, 4H, 2 x NCH₂) ; 1,64 (m, 4H, 2 x CH₂) ; 1,40 (s, 12H, 4 x CH₃) ; 1,31 (m, 2H, CH₂).

### Exemple 9: N,N'-(pentane-1,5-diylbis{(4,4-dimethyl-2,5-dioxoimidazolidine-3,1-diyl)[2-(trifluoromethyl)-4,1-phenylene]})diacetamide

Sous atmosphère d'argon, le composé de l'Exemple 8 (161 mg, 0,25 mmol) est mélangé avec du chlorure d'acétyle (10 ml) et l'agitation est maintenue pendant 15 heures à 23° C. Le mélange réactionnel est ensuite évaporé à sec (entraînement au toluène) et le résidu obtenu est purifié sur une colonne de silice (éluant : CH₂Cl₂/EtOH de 99/1 à 90/10). Après évaporation, le composé attendu est obtenu sous forme d'une mousse de couleur crème avec un rendement de 52 %.

¹H NMR 400MHz (DMSO-*d*₆) δ : 9,63 (s, 2H, CONH) ; 7,81 (d, 2H, Ph) ; 7,69 (dd, 2H, Ph) ; 7,58 (d, 2H, Ph) ; 3,30 (m, 4H, 2 x NCH₂) ; 2,06 (s, 6H, 2 x CH₃) ; 1,67 (m, 4H, 2 x CH₂) ; 1,40 (s, 12H, 4 x CH₃) ; 1,40 (m, 2H, CH₂).

### Exemple 10: 1,1'-pentane-1,5-diylbis[5,5-dimethyl-3-(3-methyl-4-nitrophenyl)-imidazolidine-2,4-dione]

### 10.1) 5,5-dimethyl-3-(3-methyl-4-nitrophenyl)imidazolidine-2,4-dione

Un mélange de 5-fluoro-2-nitrotoluene (1,55 g, 10 mmoles), 5,5-diméthylhydantoine (1,28 g, 10 mmoles), K₂CO₃ (1,38 g, 10 mmoles) dans le DMF (15 ml) est introduit dans une tube destiné au four à micro-onde et irradié à 100° C pendant 70 minutes, sous agitation magnétique. Le mélange réactionnel est ensuite versé dans de l'eau (200 ml) et extrait par AcOEt (2 x 75ml). Les phases organiques sont réunies, lavées par de la saumure, séchées sur Na₂SO₄ et filtrées. Le filtrat est concentré sous pression réduite et le résidu est purifié par chromatographie sur une colonne de silice (éluant : Heptane/AcOEt : 7/3). Le composé attendu est obtenu sous forme d'un solide blanc avec un rendement de 25 % (666 mg). Point de fusion : 177-178° C.

¹H NMR 400MHz (DMSO-*d*₆) δ : 8,70 (s, 1H, NH) ; 8,10 (d, 1H, Ph) ; 7,58 (s, 1H, Ph) ; 7,52 (dd, 1H, Ph) ; 2,54 (s, 3H, CH₃) ; 1,41 (s, 6H, 2 x CH₃).

### 10.2) 1,1'-pentane-1,5-diylbis[5,5-dimethyl-3-(3-methyl-4-nitrophenyl)-imidazolidine-2,4-dione]

Le protocole expérimental utilisé est le même que celui décrit pour la synthèse du composé de l'Exemple 2, l'intermédiaire 10.1 remplaçant le 5,5-dimethyl-3-[4-nitro-3-(trifluoromethyl)phenyl]-imidazolidine-2,4-dione. Le composé attendu est obtenu sous forme d'un solide blanc avec un rendement de 67 % (495 mg). Point de fusion : 130-131° C.

¹H NMR 400MHz (DMSO-*d*₆) δ : 8,09 (d, 2H, Ph) ; 7,58 (s, 2H, Ph) ; 7,52 (dd, 2H, Ph) ; 3,32 (s, 4H, 2 x NCH₂) ; 2,53 (s, 6H, 2 x CH₃) ; 1,68 (m, 4H, 2 x CH₂) ; 1,46 (s, 12H, 4 x CH₃) ; 1,38 (m, 2H, CH₂).

### Exemple 11 : 1,1'-pentane-1,5-diylbis{5,5-dimethyl-3-[4-nitro-2-(trifluoromethyl)-phenyl]imidazolidine-2,4-dione}

### 11.1) 5,5-dimethyl-3-[4-nitro-2-(trifluoromethyl)phenyl]imidazolidine-2,4-dione

Le protocole expérimental utilisé est le même que celui décrit pour la synthèse de l'intermédiaire 10.1, le 2-fluoro-5-nitrobenzotrifluoride remplaçant le 5-fluoro-2-nitrotoluene. Le composé attendu est obtenu sous forme d'un solide blanc avec un rendement de 29 %. Point de fusion : 175-176° C.

¹H NMR 400MHz (DMSO-*d*₆) δ : 8,76 (s, 1H, NH) ; 8,68 (dd, 1H, Ph) ; 8,58 (d, 1H, Ph) ; 8,04 (d, 1H, Ph) ; 1,47 (s, 3H, CH₃) ; 1,38 (s, 3H, CH₃).

### 11.2) 1,1'-pentane-1,5-diylbis{5,5-dimethyl-3-[4-nitro-2-(trifluoromethyl)phenyl]-imidazolidine-2,4-dione}

Le protocole expérimental utilisé est le même que celui décrit pour la synthèse du composé de l'Exemple 2, l'intermédiaire 11.1 remplaçant le 5,5-dimethyl-3-[4-nitro-3-(trifluoromethyl)phenyl]-imidazolidine-2,4-dione. Le composé attendu est obtenu sous forme d'une mousse jaune clair avec un rendement de 12 %.

¹H NMR 400MHz (DMSO-*d*₆) δ : 8,68 (m, 2H, Ph) ; 8,58 (d, 2H, Ph) ; 8,04 (d, 2H, Ph) ; 3,33 (m, 4H, 2 x NCH₂) ; 1,66 (m, 4H, 2 x CH₂) ; 1,50 (m, 6H, 2 x CH₃) ; 1,42 (m, 6H, 2 x CH₃) ; 1,35 (m, 2H, CH₂).

### Exemple 12: 1,1'-pentane-1,5-diylbis[3-(3-chloro-4-nitrophenyl)-5,5-dimethyl-imidazolidine-2,4-dione]

### 12.1) 3-(3-chloro-4-nitrophenyl)-5,5-dimethylimidazolidine-2,4-dione

Le protocole expérimental utilisé est le même que celui décrit pour la synthèse de l'intermédiaire 10.1, le 2-chloro-4-fluoronitrobenzène remplaçant le 5-fluoro-2-nitrotoluene. Le composé attendu est obtenu sous forme d'un solide jaune pâle avec un rendement de 28 %. Point de fusion : 144-145° C.

¹H NMR 400MHz (DMSO-*d*₆) δ : 8,77 (s, 1H, NH) ; 8,21 (d, 1H, Ph) ; 7,92 (d, 1H, Ph) ; 7,71 (dd, 1H, Ph) ; 1,41 (s, 6H, 2 x CH₃).

### 12.2) 1,1'-pentane-1,5-diylbis[3-(3-chloro-4-nitrophenyl)-5,5-dimethylimidazolidine-2,4-dione]

Le protocole expérimental utilisé est le même que celui décrit pour la synthèse du composé de l'Exemple 2, l'intermédiaire 12.1 remplaçant le 5,5-dimethyl-3-[4-nitro-3-(trifluoromethyl)-phenyl]-imidazolidine-2,4-dione. Le composé attendu est obtenu sous forme d'une mousse jaune clair avec un rendement de 7 %.

¹H NMR 400MHz (DMSO-*d*₆) δ : 8,28 (d, 2H, Ph) ; 7,99 (d, 2H, Ph) ; 7,79 (dd, 2H, Ph) ; 3,37 (m, 4H, 2 x NCH₂) ; 1,74 (m, 4H, 2 x CH₂) ; 1,53 (m, 12H, 4 x CH₃) ; 1,46 (m, 2H, CH₂).

### Exemple 13 : 1,1'-pentane-1,5-diylbis[3-(3-methoxy-4-nitrophenyl)-5,5-dimethylimidazolidine-2,4-dione]

### 13.1) 3-(3-methoxy-4-nitrophenyl)-5,5-dimethylimidazolidine-2,4-dione

Le protocole expérimental utilisé est le même que celui décrit pour la synthèse de l'intermédiaire 10.1, le 5-fluoro-2-nitroanisole remplaçant le 5-fluoro-2-nitrotoluene. Le composé attendu est obtenu sous forme d'un solide blanc avec un rendement de 20 %.

¹H NMR 400MHz (DMSO-*d*₆) δ : 8,70 (s, 1H, NH) ; 7,99 (d, 1H, Ph) ; 7,47 (d, 1H, Ph) ; 7,20 (dd, 1H, Ph) ; 3,91 (s, 3H, OCH₃) ; 1,42 (s, 6H, 2 x CH₃).

### 13.2) 1,1'-pentane-1,5-diylbis[3-(3-methoxy-4-nitrophenyl)-5,5-dimethylimidazolidine-2,4-dione]

Le protocole expérimental utilisé est le même que celui décrit pour la synthèse du composé de l'Exemple 2, l'intermédiaire 13.1 remplaçant le 5,5-dimethyl-3-[4-nitro-3-(trifluoromethyl)phenyl]-imidazolidine-2,4-dione. Le composé attendu est obtenu sous forme d'une mousse blanche avec un rendement de 10 %.

¹H NMR 400MHz (DMSO-*d*₆) δ : 8,07 (d, 2H, Ph) ; 7,54 (d, 2H, Ph) ; 7,28 (dd, 2H, Ph) ; 3,97 (s, 6H, 2 x OCH₃) ; 3,38 (m, 4H, 2 x NCH₂) ; 1,74 (m, 4H, 2 x CH₂) ; 1,53 (m, 12H, 4 x CH₃) ; 1,46 (m, 2H, CH₂).

### Exemple 14 : dimethyl {pentane-1,5-diylbis[(4,4-dimethyl-2,5-dioxoimidazolidine-3,1-diyl)(2-methyl-4,1-phenylene)]}biscarbamate

### 14.1) 1,1'-pentane-1,5-diylbis[3-(4-amino-3-methylphenyl)-5,5-dimethyl-imidazolidine-2,4-dione]

Le protocole expérimental utilisé est le même que celui décrit pour la synthèse du composé de l'Exemple 8 à partir du composé de l'exemple 10, celui-ci remplaçant le composé de l'exemple 2. Le composé attendu est obtenu sous forme d'une mousse blanche avec un rendement de 69 %.

¹H NMR 400MHz (DMSO-*d*₆) δ : 6,79 (m, 4H, Ph) ; 6,60 (d, 2H, Ph) ; 5,02 (s, 4H, 2 x NH₂) ; 3,26 (m, 4H, 2 x NCH₂) ; 2,03 (s, 6H, 2 x CH₃) ; 1,62 (m, 4H, 2 x CH₂) ; 1,39 (s, 12H, 4 x CH₃) ; 1,31 (m, 2H, CH₂).

### 14.2) dimethyl {pentane-1,5-diylbis[(4,4-dimethyl-2,5-dioxoimidazolidine-3,1-diyl)(2-methyl-4,1-phenylene)]}biscarbamate

Sous atmosphère d'argon, l'intermédiaire 14.1 (268 mg, 0,5 mmole) dissous dans de la pyridine anhydre (10 ml) est mélangé avec du chloroformiate de méthyle (0,8 ml, 10 mmoles). L'agitation est maintenue pendant 18 heures à 90° C. Le mélange réactionnel est ensuite versé dans de l'eau glacée et extrait à l'aide de AcOEt (2 x 50 ml). Les phases organiques sont réunies et lavées par de la saumure (25 ml). La solution organique est séchée sur Na₂SO₄, filtrée et le solvant évaporé sous pression réduite. Le résidu est purifié sur une colonne de silice (éluant : Heptane/AcOEt : 4/6 jusqu'à 0/1). Le composé attendu est obtenu sous forme d'une mousse blanche avec un rendement de 40 % (130 mg).

¹H NMR 400MHz (DMSO-*d*₆) δ : 8,92 (s, 2H, NH) ; 7,43 (d, 2H, Ph) ; 7,17 (d, 2H, Ph) ; 7,12 (dd, 2H, Ph) ; 3,66 (s, 6H, 2 x OCH₃) ; 3,29 (m, 4H, 2 x NCH₂) ; 2,20 (s, 6H, 2 x CH₃) ; 1,66 (m, 4H, 2 x CH₂) ; 1,42 (s, 12H, 4 x CH₃) ; 1,37 (m, 2H, CH₂).

### Exemple 15 : 4,4'-[pentane-1,5-diylbis(4,4-dimethyl-2,5-dioxoimidazolidine-3,1-diyl)]bis(2-methylbenzonitrile)

### 15.1) 4-(4,4-dimethyl-2,5-dioxoimidazolidin-1-yl)-2-methylbenzonitrile

Le protocole expérimental utilisé est le même que celui décrit pour la synthèse de l'intermédiaire 5.1, le 4-fluoro-2-methylbenzonitrile remplaçant le 4-fluoro-2-(trifluorométhyl)benzonitrile. Après recristallisation dans EtOH (75 ml), le composé attendu est obtenu sous forme d'un solide blanc avec un rendement de 5 %.

¹H NMR 400MHz (DMSO-*d*₆) δ : 8,70 (s, 1H, NH) ; 7,89 (d, 1H, Ph) ; 7,54 (s, 1H, Ph) ; 7,43 (d, 1H, Ph) ; 2,49 (s, 3H, CH₃) ; 1,40 (s, 6H, 2 x CH₃).

### 15.2) 4,4'-[pentane-1,5-diylbis(4,4-dimethyl-2,5-dioxoimidazolidine-3,1-diyl)]bis(2-methylbenzonitrile)

Le protocole expérimental utilisé est le même que celui décrit pour la synthèse du composé de l'Exemple 2, l'intermédiaire 15.1 remplaçant le 5,5-dimethyl-3-[4-nitro-3-(trifluoromethyl) phenyl]-imidazolidine-2,4-dione. Le composé attendu est obtenu sous forme d'un solide blanc avec un rendement de 66 %. Point de fusion : 148-149° C.

¹H NMR 400MHz (DMSO-*d*₆) δ : 7,81 (d, 2H, Ph) ; 7,49 (d, 2H, Ph) ; 7,39 (dd, 2H, Ph) ; 3,30 (m, 4H, 2 x NCH₂) ; 2,43 (s, 6H, 2 x CH₃) ; 1,62 (m, 4H, 2 x CH₂) ; 1,39 (s, 12H, 4 x CH₃) ; 1,31 (m, 2H, CH₂).

### Exemple 16 : 4,4'-[pentane-1,5-diylbis(4,4-dimethyl-2,5-dioxoimidazolidine-3,1-diyl)]bis(2-chlorobenzonitrile)

### 16.1) 2-chloro-4-(4,4-dimethyl-2,5-dioxoimidazolidin-1-yl)benzonitrile

Le protocole expérimental utilisé est le même que celui décrit pour l'intermédiaire 5.1, le 2-chloro-4-fluorobenzonitrile remplaçant le 4-fluoro-2-(trifluorométhyl)benzonitrile. Le composé attendu est obtenu sous forme d'un solide blanc avec un rendement de 23 %.

¹H NMR 400MHz (DMSO-*d*₆) δ : 8,76 (s, 1H, NH) ; 8,08 (d, 1H, Ph) ; 7,90 (d, 1H, Ph) ; 7,67 (dd, 1H, Ph) ; 1,40 (s, 6H, 2 x CH₃).

### 16.2) 4,4'-[pentane-1,5-diylbis(4,4-dimethyl-2,5-dioxoimidazolidine-3,1-diyl)]bis(2-chlorobenzonitrile)

Le protocole expérimental utilisé est le même que celui décrit pour la synthèse du composé de l'Exemple 2, l'intermédiaire 16.1 remplaçant le 5,5-dimethyl-3-[4-nitro-3-(trifluoromethyl) phenyl]-imidazolidine-2,4-dione. Le composé attendu est obtenu sous forme d'un solide blanc avec un rendement de 51 %. Point de fusion : 166-167° C.

¹H NMR 400MHz (DMSO-*d*₆) δ : 8,08 (d, 2H, Ph) ; 7,90 (d, 2H, Ph) ; 7,68 (dd, 2H, Ph) ; 3,30 (m, 4H, 2 x NCH₂) ; 1,64 (m, 4H, 2 x CH₂) ; 1,45 (s, 12H, 4 x CH₃) ; 1,38 (m, 2H, CH₂).

### Exemple 17 : 1,1'-propane-1,3-diylbis{5,5-diméthyl-3-[4-nitro-3-(trifluorométhyl)-phényl]imidazolidine-2,4-dione}

Le protocole expérimental utilisé est le même que celui décrit pour la synthèse du composé de l'Exemple 1, le 1,3-diiodopropane remplaçant le 1,4-dibromobutane. Le composé attendu est obtenu sous forme d'un solide blanc avec un rendement de 15 % (50 mg). Point de fusion : 164-165° C.

¹H NMR 400MHz (DMSO-*d*₆) δ : 8,33 (d, 2H, Ph) ; 8,21 (d, 2H, Ph) ; 8,09 (dd, 2H, Ph) ; 3,45 (t, 4H, 2 x NCH₂) ; 2,01 (m, 2H, CH₂) ; 1,50 (s, 12H, 4 x CH₃).

### Exemple 18 : chlorure de 2-{5,5-diméthyl-3-[4-nitro-3-(trifluorométhyl)phényl]-2,4-dioxoimidazolidin-1-yl}-N-(2-{5,5-diméthyl-3-[4-nitro-3-(trifluorométhyl)-phényl]-2,4-dioxoimidazolidin-1-yl}ethyl)-N-méthylethanaminium

Le protocole expérimental utilisé est le même que celui décrit pour la synthèse du composé de l'exemple 1, le chlorhydrate de méchloréthamine remplaçant le 1,4-dibromobutane. Le composé attendu est obtenu sous forme d'un solide couleur jaune clair avec un rendement de 30 %. Point de fusion : 136-137° C.

¹H NMR 400MHz (DMSO-*d*₆) δ : 11,08 (s large, 1H, NH⁺) ; 8,31 (m, 4H, Ph) ; 8,10 (d, 2H, Ph) ; 3,87 (m, 4H, 2 x NCH₂) ; 3,51 (m, 2H, NCH₂) ; 3,38 (m, 2H, NCH₂) ; 2,99 (s large, 3H, CH₃) ; 1,55 (s, 12H, 4 x CH₃).

### Exemple 19: 1,1'-(2Z)-but-2-ene-1,4-diylbis{5,5-dimethyl-3-[4-nitro-3-(trifluoromethyl) phenyl]imidazolidine-2,4-dione}

Le protocole expérimental utilisé est le même que celui décrit pour la synthèse du composé de l'exemple 1, le cis-1,4-dichloro-2-butene remplaçant le 1,4 dibromobutane. Le composé attendu est obtenu sous forme d'un solide jaune pâle avec un rendement de 40%. Point de fusion :191-193°C.

¹H NMR 400MHz (DMSO-*d*₆) δ :8,34 (d, 2H, Ph) ; 8,22 (d, 2H, Ph) ; 8,09 (dd, 2H, Ph) ; 5,61 (q, 2H, CH=CH) ; 4,2 (q, 4H, 2 x CH₂) ; 1,50 (s, 12H, 4 x CH₃).

### Exemple 20 : 4,4'-[pentane-1,5-diylbis(5,7-dioxo-4,6-diazaspiro[2.4]heptane-4,6-diyl)]bis [2-(trifluoromethyl)benzonitrile]

### 20.1) 1-({[4-cyano-3-(trifluoromethyl)phenyl]carbamoyl}amino) cyclopropanecarboxylic acid

Une solution de 4-isocyanato-2-(trifluoromethyl)benzonitrile (4,56 g, 21,5 mmol) dans de l'acétone (12 ml) est ajoutée goutte à goutte à de l'acide 1-aminocyclopropane carboxylique (2,02 g, 20 mmol) dissous dans une solution aqueuse d'hydroxyde de sodium (12ml, 0,8 g, 20 mmol). La masse réactionnelle est agitée pendant 1 heure à température ambiante puis versée sur une solution aqueuse d'hydroxyde de sodium (1N, 40 ml). La solution obtenue est lavée par de l'acétate d'éthyle (30 ml) puis acidifiée par ajout d'une solution aqueuse d'acide sulfurique (2M, 30 ml) et extraite à l'acétate d'éthyle (2 x 50 ml). La phase organique ainsi obtenue est lavée à l'eau puis par une solution aqueuse saturée de chlorure de sodium, séchée sur Na₂SO₄ et filtrée. Le solvant est évaporé sous pression réduite. Le résidu obtenu est repris par de l'éther éthylique pour conduire à un solide blanc, après filtration, avec un rendement de 50 % (3,16 g).

### 20.2) 4-(5,7-dioxo-4,6-diazaspiro[2.4]hept-6-yl)-2-(trifluoromethyl)benzonitrile

Sous argon, du chlorure d'oxalyle (1,12 ml, 13 mmol) est ajouté à une solution de l'intermédiaire 20.1 (3,16 g, 10 mmol) et de DMF (0,5 ml) dans du 1,4-dioxane (20 ml). La masse réactionnelle est agitée au reflux pendant une heure et le solvant est évaporé sous pression réduite. Le résidu est repris par de l'eau (30 ml)et la solution obtenue est extraite par de l'acétate d'éthyle (50ml). La phase organique est successivement lavée à l'eau et par une solution saturée en chlorure de sodium et séchée sur Na₂SO₄. Le solvant est évaporé sous pression réduite. Le résidu obtenu est repris par de l'éther éthylique pour conduire à un solide blanc, après filtration, avec un rendement de 20 % (0,6 g).

¹H NMR 400MHz (DMSO-*d*₆)δ :8,91 (s, 1H, NH) ; 8,27 (d, 1H, Ph) ; 8,18 (s, 1H, Ph) ; 8,04 (d, 1H, Ph) ; 1,3-1,45 (m, 4H, 2 x CH₂ spiro).

### 20.3) 4,4'-[pentane-1,5-diylbis(5,7-dioxo-4,6-diazaspiro[2.4]heptane-4,6-diyl)]bis [2-(trifluoromethyl)benzonitrile]

Le protocole expérimental utilisé est le même que celui décrit pour la synthèse du composé de l'Exemple 2, l'intermédiaire 20.2 remplaçant le 5,5-dimethyl-3-[4-nitro-3-(trifluoromethyl)-phenyl]-imidazolidine-2,4-dione. Le composé attendu est obtenu sous forme d'un solide blanc. Point de fusion : 154-155° C.

¹H NMR 400MHz (DMSO-*d*₆)δ : 8,27 (d, 2H, Ph) ; 8,17 (d, 2H, Ph) ; 8,03 (dd, 2H, Ph) ; 3,14 (t, 4H, CH₂ x 2) ; 1.64 (m, 4H, 2 x CH₂) ; 1,55(m, 4H, 2x CH₂ spiro) ; 1,36 (m, 6H, CH₂ central, 2x CH₂ spiro).

### Exemple 21 : 4,4'-[(2Z)-but-2-ene-1,4-diylbis(4,4-dimethyl-2,5-dioxoimidazolidine-3,1-diyl)]bis[2-(trifluoromethyl)benzonitrile]

Le protocole expérimental utilisé est le même que celui décrit pour la synthèse de l'exemple 1, le cis-1,4-dichloro-2-butene remplaçant le 1,4 dibromobutane et l'intermédiaire 5.1 remplaçant le 5,5-dimethyl-3-[4-nitro-3-(trifluoromethyl)-phenyl]-imidazolidine-2,4-dione. Le composé attendu est obtenu sous forme d'un solide beige clair. Point de fusion :188-190°C.

¹H NMR 400MHz (DMSO-d₆) δ :8,32 (d, 2H, Ph) ; 8,19 (d, 2H, Ph) ; 8,05 (dd, 2H, Ph) ; 5,60 (q, 2H, CH=CH) ; 4,2 (q, 4H, 2 x CH₂) ; 1,49 (s, 12H, 4 x CH₃).

### Exemple 22 : 4,4'-{(2R,3S)-oxirane-2,3-diylbis[methanediyl(4,4-dimethyl-2,5-dioxoimidazolidine-3,1-diyl)]}bis[2-(trifluoromethyl)benzonitrile]

Sous argon, le composé de l'exemple 21 (90 mg, 0.139 mmol) est mélangé avec de l'acide 3-métachloroperbenzoïque (72.5 mg, 0.210 mmol) dans du dichlorométhane anhydre (10 ml). Le mélange est agité à température ambiante. L'avancement de la réaction est suivie par CCM (éluant DCM/EtOH : 95/05). Après 4 jours, il reste du composé de départ. Une nouvelle quantité d'acide métachloroperbenzoïque (0,124 g, 0.36 mmol) est ajoutée à la masse réactionnelle et la réaction est agitée à température ambiante pendant 4 jours supplémentaires. Le solvant est évaporé sous pression réduite et le résidu est purifié par chromatographie flash (colonne BIOTAGE 25+M, gradient dichloromethane/acétone : 0% à 8% en acétone). Le composé attendu est obtenu sous la forme d'un solide blanc, avec un rendement de 50%. Point de fusion : 108-110°C.

¹H NMR 400MHz (DMSO-*d*₆)δ.:8,32 (d, 2H, Ph) ; 8,21 (d, 2H, Ph) ; 8,05 (dd, 2H, Ph) ; 4,05 (q, 2H, CH-CH) ; 3.28 (m, 4H, 2 x CH₂) ; 1,53(d, 12H, 4 x CH₃).

### Exemple 23 : 4,4'-{(1R,2R)-cyclopropane-1,2-diylbis[methanediyl(4,4-dimethyl-2,5-dioxoimidazolidine-3,1-diyl)]}bis[2-(trifluoromethyl)benzonitrile]

Le protocole expérimental utilisé est le même que celui décrit pour la synthèse du composé de l'Exemple 1, le trans-1,2-bis(bromomethyl)cyclopropane ( préparé selon J. Med. Chem. 2003, 46 (21), 4586-4600) remplaçant le 1,4-dibromobutane. Le composé attendu est obtenu sous forme d'un solide de couleur crème. Point de fusion : 178-180°C.

¹H NMR 400MHz (DMSO-*d*₆)δ : 8,26 (d, 2H, Ph) ; 8,15 (d, 2H, Ph) ; 8,01 (dd, 2H, Ph) ; 3,2-3,4 (m, 4H, 2 x NCH₂) ; 2,49 (s, 12H, 4 x CH₃) ; 1,24 (m, 2H, 2 x CH) ; 0,61 (t, 2H, 1 x CH₂).

### Etude pharmacologigue des composés de l'invention

### Mesures des activités anti-prolifératives :

### 1. Activité anti-proliférative sur LNCaP en milieu complet

L'activité anti-proliférative des composés de la présente invention est déterminée sur LNCaP en milieu complet en appliquant la procédure expérimentale suivante :

Le type cellulaire LNCaP (ATCC, 1740) est issu d'un carcinome de prostate exprimant le récepteur aux androgènes, elle est hormono-dépendante.

L'entretien de la lignée LNCaP est réalisé en milieu de culture complet : RPMI, 10 % de sérum de veau foetal, 2 mM glutamine, 100 U/ml pénicilline, 0,1 mg/ml de streptomycine et HEPES 0,01 M, pyruvate de sodium 1 mM , 40 % de D-glucose.
- Ensemencement des plaques :
   La lignée LNCaP est ensemencée à 20000 cellules/puits dans 90 µl de milieu complet en plaques 96 puits coatées poly-D-lysine (Biocoat , Costar).
- Traitement des cellules : 24h après l'ensemencement, les cellules sont traitées avec 10 µl/puits de composé dilué dans le milieu de culture. Les concentrations utilisées sont les suivantes : 1/10/30/100/300/1000/3000/10000/100000 nM. Les cellules sont incubées 144 h à 37° C, 5 % CO₂.
- Lecture : Après 6 jours d'incubation, 10 µL du réactif "cell prolifération WST-1" (Roche ref 1644807) est ajouté dans chaque puits. Après une incubation de 2 heures à 37° C, 5 % CO₂, l'absorbance à 450 nm est mesurée par spectrophotométrie (Envision, Perkin Elmer).
- Résultats : Les expériences sont réalisées en duplicat et les meilleurs composés sont testés deux fois. Une valeur de concentration inhibant de 50 % la prolifération cellulaire (CI₅₀) est calculée.
   Tous les composés des exemples 1 à 23 décrits précédemment ont des CI₅₀ inférieures ou égales à 5000 nM sur les cellules LNCaP en culture.
   Parmi eux, les composés des exemples suivants ont des CI₅₀ inférieures ou égales à 1500 nM sur les cellules LNCaP en culture : 1, 2, 4, 7, 10, 13, 14, 15, 16, 19, 20, 21 et 22.
   Les composés des exemples suivants ont des CI₅₀ inférieures ou égales à 500 nM sur les cellules LNCaP en culture : 1, 2, 7, 15, 19, 20, 21 et 22.

### 2. Activité antiproliférative sur LNCaP en milieu déplété en stéroïde :

L'activité pro et/ou anti proliférative des composés de la présente invention est déterminée sur LNCaP en milieu déplété en stéroïde.

La lignée LNCaP (ATCC, 1740) est issue d'un carcinome de prostate exprimant le récepteur aux androgènes, elle est hormono-dépendante.

L'entretien de la lignée LNCaP est réalisé dans les conditions habituelles en RPMI, 10 % de sérum de veau foetal, 2 mM de glutamine, 100 U/ml de pénicilline, 0,1 mg/ml de streptomycine et HEPES 0,01 M, pyruvate de sodium 1 mM, 40 % de D-glucose.

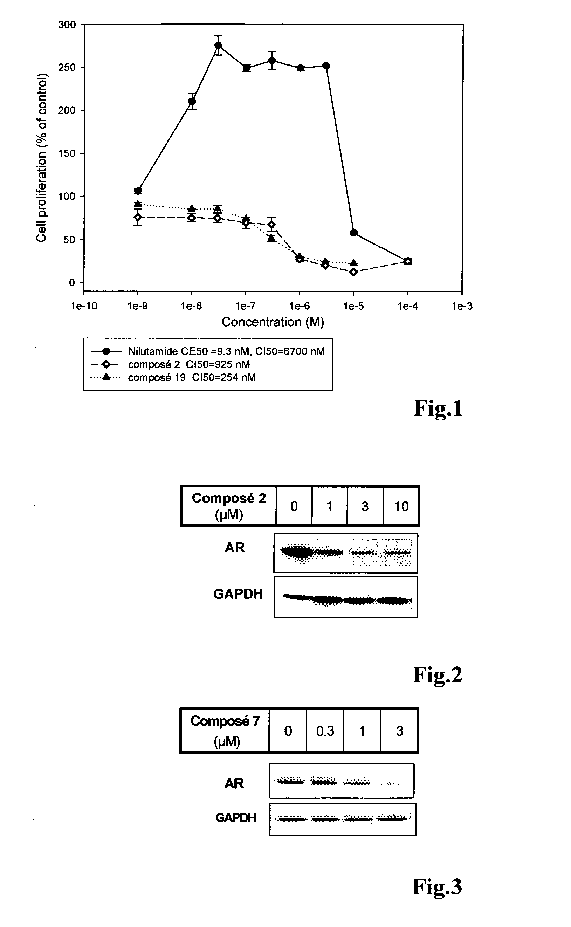

Pour l'étude en conditions sans stéroïde, 24 heures avant l'ensemencement le milieu de culture des cellules est éliminé. Les cellules sont lavées avec du PBS, puis incubées en présence de milieu RPMI sans rouge de phénol, 10 % de sérum de veau foetal déplété en stéroïde (traitement préalable au charbon-dextran), 2 mM de glutamine, 100 U/ml de pénicilline, 0,1 mg/ml de streptomycine et HEPES 0,01M, pyruvate de sodium 1 mM, 40 % de D-glucose.
- Ensemencement des plaques :
   La lignée LNCaP est ensemencée à 20000 cellules/puits dans 90 µl de milieu RPMI avec 10% de serum de veau foetal déplété en stéroïde en plaques 96 puits coatées poly-D-lysine (Biocoat, Costar).
- Traitement des cellules : 24h après l'ensemencement, les cellules sont traitées avec 10 (µl/puits de composé dilué dans le milieu de culture. Les concentrations utilisées sont les suivantes : 1/10/30/100/300/1000/3000/10000/100000 nM. Les cellules sont incubées 144 h à 37° C, 5 % CO₂.
- Lecture : Après 6 jours d'incubation, 10 µL de réactif "cell prolifération WST-1" (Roche ref 1644807) est ajouté dans chaque puits. Après une incubation de 2 à 4 heures à 37° C, 5 % CO₂, l'absorbance à 450 nm est mesurée par spectrophotométrie (Envision, Perkin Elmer).
- Résultats : Les expériences sont réalisées en duplicat et les meilleurs composés sont testés deux fois. Une valeur de concentration inhibant de 50 % la prolifération cellulaire (CI₅₀) est calculée.
   Comme décrit précédemment (Veldscholte J, Berrevoets CA, Brinkmann AO, Grootegoed JA, Mulder E.Biochemistry 1992 Mar 3;31(8):2393-9), le nilutamide présente une activité agoniste aux faibles concentrations, puis une activité inhibitrice aux fortes concentrations.
   De façon surprenante, les composés 1 à 23 ne présentent pas d'effet agoniste sur les LNCaP cultivées en milieu déplété en stéroïde. Les composés 2 et 19 montrent de plus une activité inhibitrice visible aux faibles concentrations.
   L'effet des composés 2 et 19 sur la prolifération cellulaire des LNCaP cultivées en milieu déplété en stéroïde est présenté dans la figure 1.

### 3. Mesure de l'expression protéique du récepteur aux androgènes

Les cellules de la lignée LNCaP sont ensemencées à raison de 2,5 millions de cellules en boite de Pétri 10cm en RPMI, 10 % de sérum de veau foetal, 2 mM de glutamine, 100 U/ml de pénicilline, 0,1 mg/ml de streptomycine et HEPES 0,01 M, pyruvate de sodium 1 mM, 40 % de D-glucose. 4 jours après, les cellules sont traitées par le composé à tester. 72 heures après le traitement les cellules sont lysées en tampon de lyse (Tris pH7.4 50 mM, NaCl 150 mM, EDTA 1 mM, NaF 20 mM, Na₂VO₃ 100 mM, NP40 0.5%, Triton X-100 1%, EGTA 1mM, Pefabloc, protéase inhibiteur cocktail 11836170001 RocheDiagnostics, Phosphatase inhibitor cocktail set II Calbiochem). Les cellules sont ensuite grattées et le lysat transféré en tubes QIAshredder (cat n°79656 Qiagen) pour centrifugation à 13 000 rpm pendant 15 min à 4°C. Le surnageant est transféré dans les tubes QIAshredder pour une seconde centrifugation à 13000 rpm pendant 5 min afin d'éliminer complètement les filaments d'ADN. La concentration protéique est alors déterminée (Bio-Rad DC protein assay kit) et ajustée de façon à charger la même quantité de protéines par puit (10 et 20µg par puits selon les expériences). Le tampon de charge (sample loading buffer 3 X ref 7722 Cell signaling technology) additionné de beta-mercaptoéthanol à 1% et de DTT à 50 mM est ajouté aux échantillons qui sont ensuite chauffés pendant 10 min à 90°C. Les échantillons sont déposés sous un volume de 20 µl sur des gels NuPAGE 4-12% Bis-Tris gel (cat N° NP0322BOX, Invitrogen). La migration a lieu en tampon MOPS (Invitrogen) et s'effectue pendant 1 heure à 180V. Les protéines sont transférées sur une membrane de nitrocellulose (Hybond ECL RPN78D, GE Healthcare) dans des conditions semi-sèches, en présence de tampon de transfert (NP0006-1, Invitrogen) pendant 45 min à 15V. La membrane est ensuite bloquée pendant 1 heure dans du tampon de blocage (Non-fat dry milk, cat 170-6404, Biorad) à 5% dans du tampon Tris Buffer saline (TBS) 0.1% Tween 20. Puis elle est incubée à 4°C toute la nuit en présence d'anticorps primaire dirigé contre le récepteur à l'androgène (AR441, sc-7305, Santa Cruz) dilué au 1/2000^{ème} dans du tampon de blocage ainsi qu'en présence d'anticorps primaire dirigé contre la GAPDH (Cat.MAB374, Millipore) dilué au 1/20000^{ème} dans du tampon de blocage (contrôle de charge en protéines). La membrane est ensuite lavée 3 fois dans du tampon de lavage (TBS, 0.1 % Tween 20). La membrane est ensuite incubée en présence d'anticorps secondaire anti-immunoglobuline de souris couplée à l'HRP (Goat anti-mouse IgG-HRP, sc 2031, Santa Cruz) dilué au 1/5000^{ème} dans du tampon de blocage. La membrane est ensuite lavée 3 fois dans du tampon de lavage. Les protéines sont révélées par électrochimioluminescence (western Blotting detection system ECL+, Amersham) qui est détectée soit à l'aide de films photographiques (Biomax light, Sigma), soit par un système d'acquisition de la chimioluminescence (G :Box, Syngene).

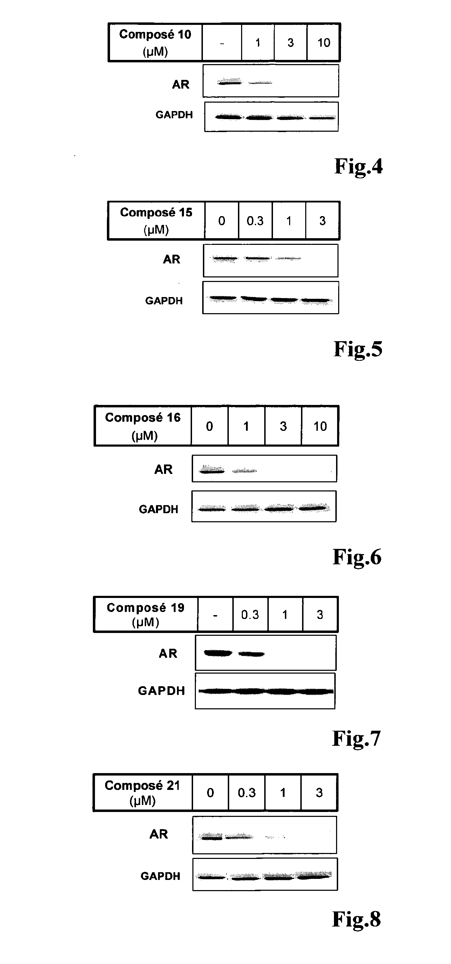

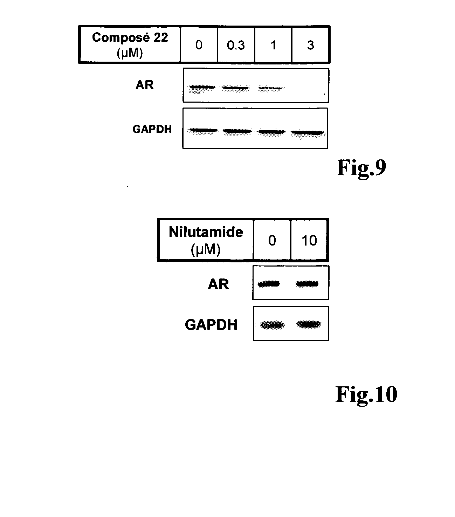

Les effets des composés 2, 7, 10, 15, 16, 19, 21, 22 sont présentés Figure 2 à 9 : ces composés diminuent l'expression protéique du récepteur aux androgènes. En revanche, comme le montre la figure 10, le nilutamide ne diminue pas l'expression protéique de ce récepteur (figure 10).

## Revendications

1. Composé de formule générale **(I)** 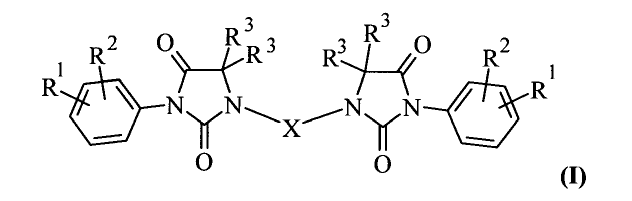 dans laquelle :
R¹ représente un radical cyano, nitro, amino, -NHCOOR⁴ ou -NHCOR⁴ ;
R² représente un radical halo, alkyle, haloalkyle, ou alkoxy ;
R³ représente un radical alkyle ou un atome d'hydrogène ; ou bien les deux radicaux R³ forment ensemble avec l'atome de carbone auquel ils se rattachent un cycloalkyle comportant de 3 à 4 chaînons ;
X représente
• soit une chaîne alkylène de 3 à 7 atomes de carbone, linéaire ou ramifiée, cette chaîne pouvant contenir un ou plusieurs chaînons identiques ou différents supplémentaires choisis parmi -O-, -N(R⁵)-, -S-, -SO- ou -SO₂- ; ou
• soit un groupe 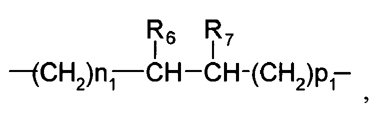 où n1 et p1 sont deux entiers dont la somme n1 + p1 est un nombre entier choisi parmi 2, 3, 4 ou 5 ;
R⁶ et R⁷ forment ensemble une liaison covalente, ou R⁶ et R⁷ forment ensemble avec les atomes de carbone auxquels ils sont reliés le cycle 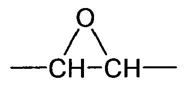 ou un cycloalkyle comportant de 3 à 6 chaînons ;
R⁴ représente un radical alkyle, aryle, ou hétéroaryle ;
R⁵ représente un hydrogène, un radical alkyle, ou aralkyle ;
ou un sel pharmaceutiquement acceptable de ce dernier.

2. Composé selon la revendication 1, dans lequel X représente une chaîne alkylène de 3 à 7 atomes de carbone, linéaire ou ramifiée, cette chaîne pouvant contenir un ou plusieurs chaînons identiques ou différents supplémentaires choisis parmi -O-, -N(R⁵)-, -S-, -SO- ou -SO₂- ou un sel pharmaceutiquement acceptable de ce dernier.

3. Composé selon la revendication 1 ou 2, dans lequel X représente une chaîne alkylène pouvant contenir un seul chaînon choisi parmi -O-, -N(R⁵)-, -S-, -SO- ou -SO₂- ou un sel pharmaceutiquement acceptable de ce dernier.

4. Composé selon la revendication 1, dans lequel X représente un groupe 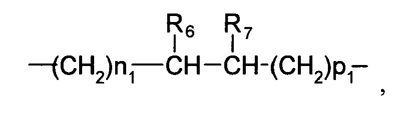 où n1 et p1 sont deux entiers dont la somme n1 + p1 est un nombre entier choisi parmi 2, 3, 4 et 5 ;
R⁶ et R⁷ forment ensemble une liaison covalente, ou R⁶ et R⁷ forment ensemble avec les atomes de carbone auquels ils sont reliés le cycle 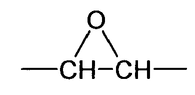 ou un cycloalkyle comportant de 3 à 4 chaînons ; ou un sel pharmaceutiquement acceptable de ce dernier.

5. Composé selon la revendication 4, dans lequel n1 et p1 sont égaux.

6. Composé selon la revendication 1, dans lequel X représente un groupe (̵CH₂)n₂-X'-(CH₂)p₂, et X' représente un groupement -O-, -N(R⁵)- ou -S-, -SO-, -SO₂-, -(CH₂)- ou 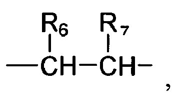 et n2 et p2 sont des entiers dont la somme n2 + p2 est soit un nombre entier choisi parmi 3, 4, 5, 6 et 7 lorsque X' représente un groupement -O-, -N(R⁵)- ou -S-, -SO-, -SO₂- soit un nombre entier choisi parmi 2, 3, 4 et 5 lorsque X' représente un groupement 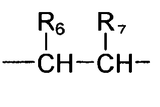 ou -(CH₂)-.

7. Composé selon la revendication 6, dans lequel X' représente un groupe

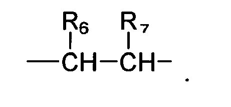

8. Composé selon la revendication 6, dans lequel X' représente un groupement -O-, - N(R⁵)- ou -(CH₂)-.

9. Composé selon l'une quelconque des revendications 6 à 8, dans lequel n2 et p2 sont égaux.

10. Composé selon l'une des revendications 1 à 9, dans lequel R³ représente un radical alkyle ou les deux radicaux R³ forment ensemble avec l'atome de carbone auquel ils se rattachent un cycloalkyle comportant de 3 à 4 chaînons.

11. Composé selon l'une des revendications 1 à 10, dans lequel R⁴ représente un radical alkyle.

12. Composé selon l'une des revendications 1 à 11, dans lequel R⁵ représente un radical alkyle.

13. Composé selon l'une des revendications 1 à 12, dans lequel R¹ est en position para.

14. Composé selon l'une des revendications 1 à 13, dans lequel R² est en position meta.

15. Composé selon l'une des revendications 1 à 14, dans lequel R⁶ et R⁷ forment ensemble une liaison covalente.

16. Composé selon l'une des revendications 1 à 14, dans lequel R⁶ et R⁷ forment ensemble avec les atomes de carbone auxquels ils sont reliés le cycle

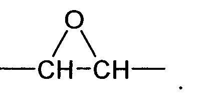

17. Composé selon l'une des revendications 1 à 14, dans lequel R⁶ et R⁷ forment ensemble avec les atomes de carbone auxquels ils sont reliés un cycloalkyle comportant de 3 à 6 chaînons.

18. Composé selon l'une des revendications 1 à 17, dans lequel le radical alkyle représente un groupement méthyle.

19. Composé de formule générale **(I)** choisi parmi :
- 1,1'-butane-1,4-diylbis{5,5-dimethyl-3-[4-nitro-3-(trifluoromethyl)-phenyl]imidazolidine-2,4-dione}
- 1,1'-pentane-1,5-diylbis{5,5-dimethyl-3-[4-nitro-3-(trifluoromethyl)-phenyl]imidazolidine-2,4-dione}
- 1,1'-(oxydiethane-2,1-diyl)bis{5,5-dimethyl-3-[4-nitro-3-(trifluoromethyl)-phenyl]imidazolidine-2,4-dione}
- 4,4'-[pentane-1,5-diylbis(4,4-dimethyl-2,5-dioxoimidazolidine-3,1 -diyl)]bis(2-methylbenzonitrile)
- 1,1'-(2Z)-but-2-ene-1,4-diylbis{5,5-dimethyl-3-[4-nitro-3-(trifluoromethyl) phenyl]imidazolidine-2,4-dione}
ou un sel pharmaceutiquement acceptable de celui-ci

20. Composé de formule générale **(I)**, consistant en le composé 1,1'-pentane-1,5-diylbis{5,5-dimethyl-3-[4-nitro-3-(trifluoromethyl)-phenyl]imidazolidine-2,4-dione}ou du composé 1,1'-(2Z)-but-2-ene-1,4-diylbis{5,5-dimethyl-3-[4-nitro-3-(trifluoromethyl) phenyl]imidazolidine-2,4-dione} ou un sel pharmaceutiquement acceptable de ces derniers.

21. Procédé de préparation d'un composé de formule **(I)** tel que défini à la revendication 1, comprenant une étape qui consiste à
(i) condenser deux équivalents d'arylhydantoïnes de formule générale **(II)** 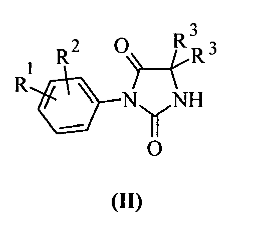 dans laquelle R² et R³ sont tels que définis à la revendication 1 et R¹ est un groupement nitro ou cyano, sur un dérivé de formule générale Gp¹-X-Gp², Gp¹ et Gp² étant des groupes partants, et X étant tel que défini précédemment, en présence d'une base forte, pour former le composé de formule générale **(I)** 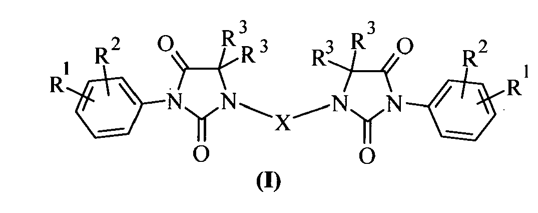 dans laquelle R², R³ et X sont tels que définis à la revendication 1 et R¹ est un groupement nitro ou cyano ;
optionnellement, si R¹ est un groupement nitro, le procédé peut comprendre en outre une étape de :
(ii) réduction du groupement nitro afin d'obtenir un composé de formule (III) 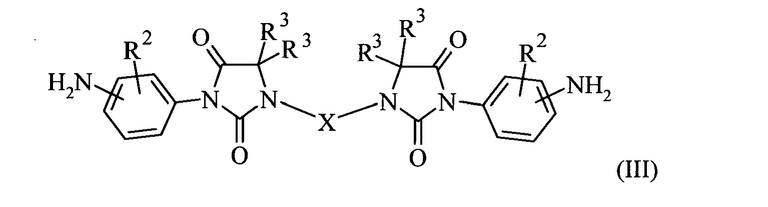 optionnellement, le procédé peut comprendre en outre une étape choisie parmi :
(iii) la réaction d'un composé de formule (III) obtenu dans l'étape (ii) avec un chlorure d'acide de formule générale R⁴-COCl, dans lequel R⁴ est tel que défini dans la revendication 1, afin d'obtenir un composé de formule (IV) 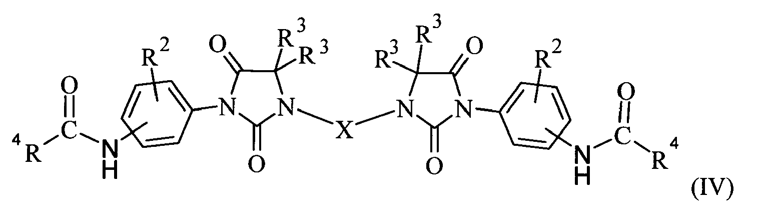
(iv) la réaction d'un composé de formule (III) obtenu dans l'étape (ii) avec un chloroformiate de formule générale R⁴-O-CO-Cl dans lequel R⁴ est tel que défini dans la revendication 1 afin d'obtenir un composé de formule (V) 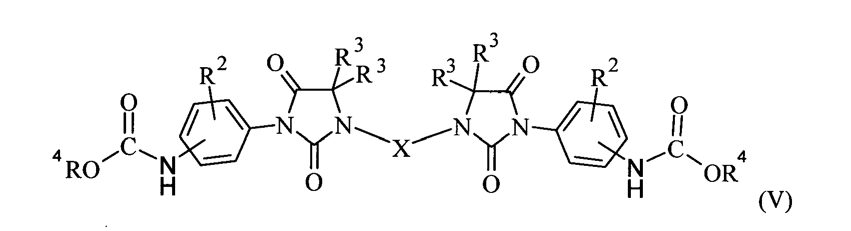 optionnellement, si R⁶ et R⁷ forment ensemble une liaison covalente, le procédé peut comprendre en outre une étape de :
(v) oxydation du composé de formule (I) dans lequel R⁶ et R⁷ forment ensemble une liaison covalente, de la double liaison ainsi formée par R⁶ et R⁷ afin d'obtenir un composé de formule (VI)

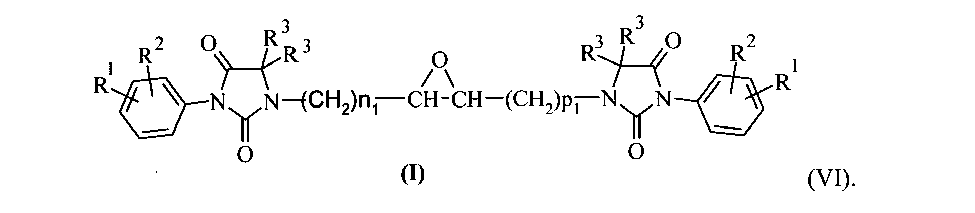

22. En tant que médicament, un composé selon d'une des revendications 1 à 20.

23. Compositions pharmaceutiques contenant, à titre de principe actif, au moins un composé de formule **(I)** tel que défini à l'une des revendications 1 à 20, en association avec un support pharmaceutiquement acceptable.

24. Utilisation d'un composé de formule **(I)** selon l'une des revendications 1 à 20, pour la préparation d'un médicament destiné à traiter les cancers.

25. Utilisation selon la revendication 24, dans laquelle le médicament est destiné à traiter un cancer hormono-dépendant.

26. Utilisation selon la revendication 24 dans laquelle le médicament est destiné à traiter un cancer exprimant les récepteurs aux androgènes

27. Utilisation selon l'une des revendications 24 à 26 dans laquelle le médicament est destiné à traiter un cancer du sein ou de la prostate.

## Patentansprüche

1. Verbindung der allgemeinen Formel (I) 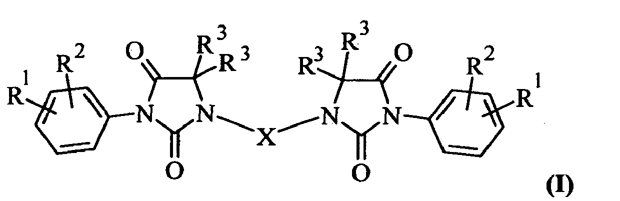 in der:
R¹ einen Cyano-, Nitro-, Amino-, -NHCOOR⁴- oder -NHCOR⁴-Rest darstellt;
R² einen Halogen-, Alkyl-, Halogenalkyl- oder Alkoxyrest darstellt;
R³ einen Alkylrest oder ein Wasserstoffatom darstellt, oder die zwei Reste R³ zusammen mit dem Kohlenstoffatom, an das sie gebunden sind, ein Cycloalkyl, das 3 bis 4 Kettenglieder umfasst, bilden;
X darstellt:
• eine lineare oder verzweigte Alkylenkette mit 3 bis 7 Kohlenstoffatomen, wobei diese Kette ein oder mehrere gleiche oder unterschiedliche zusätzliche Kettenglieder, ausgewählt aus -O-, -N(R⁵)-, -S-, -SO- oder -SO₂-, enthalten kann, oder
• eine Gruppe 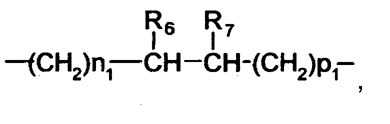 worin n1 und p1 zwei ganze Zahlen sind, deren Summe n1 + p1 eine ganze Zahl, ausgewählt aus 2, 3, 4 oder 5, ist;
R⁶ und R⁷ zusammen eine kovalente Bindung bilden oder R⁶ und R⁷ zusammen mit den Kohlenstoffatomen, an die sie gebunden sind,
den Ring 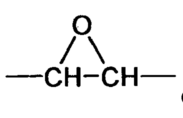 oder ein Cycloalkyl, das 3 bis 6 Kettenglieder umfasst, bilden;
R⁴ einen Alkyl-, Aryl- oder Heteroarylrest darstellt;
R⁵ einen Wasserstoff, einen Alkyl- oder Aralkylrest darstellt;
oder ein pharmazeutisch verträgliches Salz davon.

2. Verbindung gemäß Anspruch 1, wobei X eine lineare oder verzweigte Alkylenkette mit 3 bis 7 Kohlenstoffatomen darstellt, wobei diese Kette ein oder mehrere gleiche oder verschiedene zusätzliche Kettenglieder, ausgewählt aus -O-, -N(R⁵)-, -S-, -SO- oder -SO₂-, enthalten kann, oder ein pharmazeutisch verträgliches Salz davon.

3. Verbindung gemäß Anspruch 1 oder 2, wobei X eine Alkylenkette darstellt, die ein einzelnes Kettenglied, ausgewählt aus -O-, -R(R⁵)-, -S-, -SO- oder -SO₂-, enthalten kann, oder ein pharmazeutisch verträgliches Salz davon.

4. Verbindung gemäß Anspruch 1, wobei X eine Gruppe 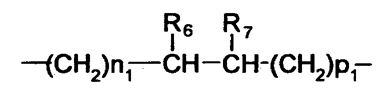 darstellt, in der n1 und p1 zwei ganze Zahlen sind, deren Summe n1 + p1 eine ganze Zahl, ausgewählt aus 2, 3, 4 und 5, ist;
R⁶ und R⁷ zusammen eine kovalente Bindung bilden oder R⁶ und R⁷ zusammen mit den Kohlenstoffatomen, an die sie gebunden sind, den Ring 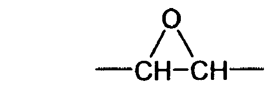 oder ein Cycloalkyl, das 3 bis 4 Kettenglieder umfasst, bilden; oder ein pharmazeutisch verträgliches Salz davon.

5. Verbindung gemäß Anspruch 4, wobei n1 und p1 gleich sind.

6. Verbindung gemäß Anspruch 1, wobei X eine Gruppe -(CH₂)n₂-X'-(CH₂)p₂ darstellt und X' eine Gruppierung -O-, -N(R⁵)- oder -S-, -SO-, -SO₂-, -(CH₂)-oder 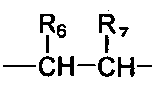 darstellt, und n2 und p2 ganze Zahlen sind, deren Summe n2 + p2 entweder eine ganze Zahl, ausgewählt aus 3, 4, 5, 6 und 7, wenn X' eine Gruppierung -O-, -N(R⁵)- oder -S-, -SO-, -SO₂- darstellt, oder eine ganze Zahl, ausgewählt aus 2, 3, 4 und 5, wenn X' eine Gruppierung 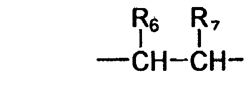 oder -(CH₂)- darstellt, ist.

7. Verbindung gemäß Anspruch 6, wobei X' eine Gruppe 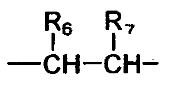 darstellt.

8. Verbindung gemäß Anspruch 6, wobei X' eine Gruppierung -O-, -N(R⁵)-oder -(CH₂)- darstellt.

9. Verbindung gemäß einem der Ansprüche 6 bis 8, wobei n2 und p2 gleich sind.

10. Verbindung gemäß einem der Ansprüche 1 bis 9, wobei R³ einen Alkylrest darstellt oder die zwei Reste R³ zusammen mit dem Kohlenstoffatom, an das sie gebunden sind, ein Cycloalkyl, das 3 bis 4 Kettenglieder umfasst, bilden.

11. Verbindung gemäß einem der Ansprüche 1 bis 10, wobei R⁴ einen Alkylrest darstellt.

12. Verbindung gemäß einem der Ansprüche 1 bis 11, wobei R⁵ einen Alkylrest darstellt.

13. Verbindung gemäß einem der Ansprüche 1 bis 12, wobei R¹ in para-Position ist.

14. Verbindung gemäß einem der Ansprüche 1 bis 13, wobei R² in meta-Position ist.

15. Verbindung gemäß einem der Ansprüche 1 bis 14, wobei R⁶ und R⁷ zusammen eine kovalente Bindung bilden.

16. Verbindung gemäß einem der Ansprüche 1 bis 14, wobei R⁶ und R⁷ zusammen mit den Kohlenstoffatomen, an die sie gebunden sind, den Ring 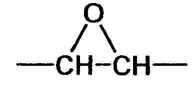 bilden.

17. Verbindung gemäß einem der Ansprüche 1 bis 14, wobei R⁶ und R⁷ zusammen mit den Kohlenstoffatomen, an die sie gebunden sind, ein Cycloalkyl, das 3 bis 6 Kettenglieder umfasst, bilden.

18. Verbindung gemäß einem der Ansprüche 1 bis 17, wobei der Alkylrest eine Methyl-Gruppierung darstellt.

19. Verbindung der allgemeinen Formel (I), ausgewählt aus:
- 1,1'-Butan-1,4-diylbis{5,5-dimethyl-3-[4-nitro-3-(trifluormethyl)-phenyl]imidazolidin-2,4-dion}
- 1,1'-Pentan-1,5-diylbis{5,5-dimethyl-3-[4-nitro-3-(trifluormethyl)-phenyl]imidazolidin-2,4-dion}
- 1,1'-(Oxydiethan-2,1-diyl)bis{5,5-dimethyl-3-[4-nitro-3-(trifluormethyl)-phenyl]imidazolidin-2,4-dion}
- 4,4'-[Pentan-1,5-diylbis(4,4-dimethyl-2,5-dioxoimidazolidin-3,1 -diol)]-bis(2-methylbenzonitril)
- 1,1'-(2Z)-But-2-en-1,4-diylbis{5,5-dimethyl-3-[4-nitro-3-(trifluormethyl)phenyl]imidazolidin-2,4-dion)
oder ein pharmazeutisch verträgliches Salz davon.

20. Verbindung der allgemeinen Formel (I), die aus der Verbindung 1,1'-Pentan-1,5-diylbis{5,5-dimethyl-3-[4-nitro-3-(trifluormethyl)-phenyl]-imidazolidin-2,4-dion} oder der Verbindung 1,1'-(2Z)-But-2-en-1,4-diylbis{5,5-dimethyl-3-[4-nitro-3-(trifluormethyl)phenyl]imidazolidin-2,4-dion} besteht, oder ein pharmazeutisch verträgliches Salz derselben.

21. Verfahren zur Herstellung einer Verbindung der Formel (I), wie sie in Anspruch 1 definiert ist, umfassend eine Stufe, bestehend aus
(i) Kondensation von zwei Äquivalenten von Arylhydantoinen der allgemeinen Formel (II) 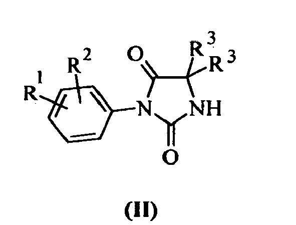 in der R² und R³ wie in Anspruch 1 definiert sind und R¹ eine Nitro- oder Cyano-Gruppierung ist, mit einem Derivat der allgemeinen Formel Gp¹-X-Gp², wobei Gp¹ und Gp² Abgangsgruppen sind und X wie vorstehend definiert ist, in Gegenwart einer starken Base unter Bildung der Verbindung der allgemeinen Formel (I) 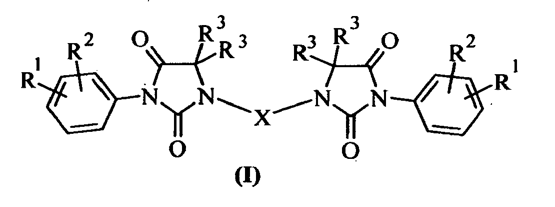 in der R², R³ und X wie in Anspruch 1 definiert sind und R¹ eine Nitro- oder Cyano-Gruppierung ist;
wobei das Verfahren gegebenenfalls, wenn R¹ eine Nitro-Gruppierung ist, außerdem folgende Stufe umfassen kann:
(ii) Reduktion der Nitro-Gruppierung, um eine Verbindung der Formel (III) zu erhalten 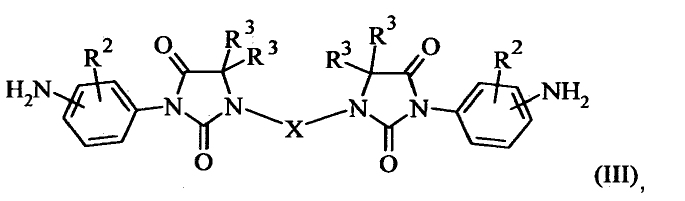 wobei das Verfahren gegebenenfalls außerdem eine Stufe umfassen kann, die ausgewählt ist aus:
(iii) Reaktion einer Verbindung der in Stufe (ii) erhaltenen Verbindung der Formel (III) mit einem Säurechlorid der allgemeinen Formel R⁴-COCl, in der R⁴ wie in Anspruch 1 definiert ist, unter Erhalt einer Verbindung der Formel (IV) 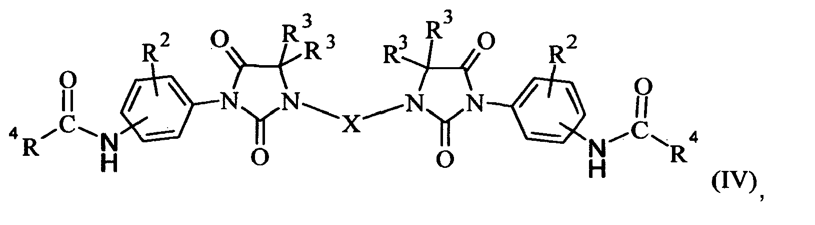
(iv) Reaktion einer in Stufe (ii) erhaltenen Verbindung der Formel (III) mit einem Chlorformiat der allgemeinen Formel R⁴-O-CO-Cl, in der R⁴ wie in Anspruch 1 definiert ist, unter Erhalt einer Verbindung der Formel (V) 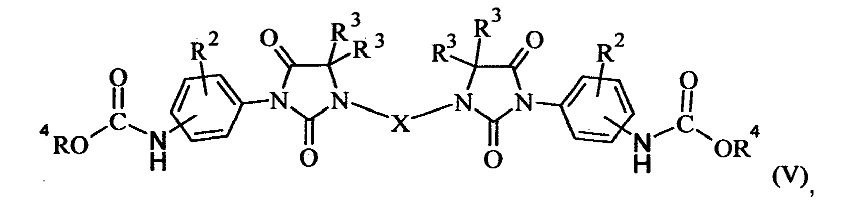 wobei das Verfahren gegebenenfalls, wenn R⁶ und R⁷ zusammen eine kovalente Bindung bilden, außerdem folgende Stufe umfassen kann:
(v) Oxidation in der Verbindung der Formel (I), in der R⁶ und R⁷ zusammen eine kovalente Bindung bilden, der so durch R⁶ und R⁷ gebildeten Doppelbindung unter Erhalt einer Verbindung der Formel (VI)

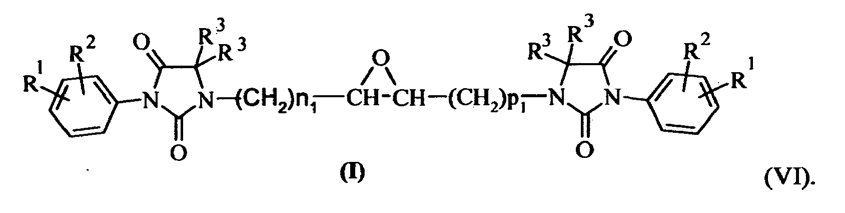

22. Verbindung gemäß einem der Ansprüche 1 bis 20 als Medikament.

23. Pharmazeutische Zusammensetzungen, die als Wirkstoff wenigstens eine Verbindung der Formel (I), wie in einem der Ansprüche 1 bis 20 definiert, in Verbindung mit einem pharmazeutisch verträglichen Träger enthalten.

24. Verwendung einer Verbindung der Formel (I) gemäß einem der Ansprüche 1 bis 20 für die Herstellung eines Medikaments, das dazu bestimmt ist, Krebsarten zu behandeln.

25. Verwendung gemäß Anspruch 24, wobei das Medikament dazu bestimmt ist, einen hormonabhängigen Krebs zu behandeln.

26. Verwendung gemäß Anspruch 24, wobei das Medikament dazu bestimmt ist, einen Krebs zu behandeln, der Rezeptoren für Androgene exprimiert.

27. Verwendung gemäß einem der Ansprüche 24 bis 26, wobei das Medikament dazu bestimmt ist, Brustkrebs oder Prostatakrebs zu behandeln.

## Claims

1. Compound of general formula **(I)** 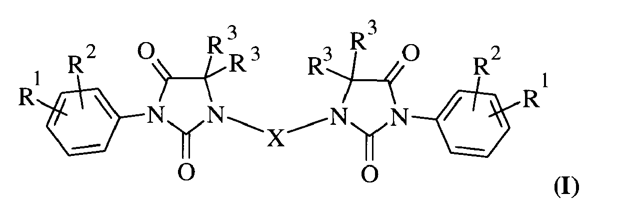 in which:
R¹ represents a cyano, nitro, amino, -NHCOOR⁴ or -NHCOR⁴ radical;
R² represents a halo, alkyl, haloalkyl, or alkoxy radical;
R³ represents an alkyl radical or a hydrogen atom; or the two R³ radicals form together with the carbon atom to which they are attached a cycloalkyl comprising 3 to 4 members;
X represents
• either a linear or branched alkylene chain with 3 to 7 carbon atoms, this chain being able to contain one or more additional identical or different members chosen from -O-, -N(R⁵)-, -S-, -SO- or -SO₂-;
• or a 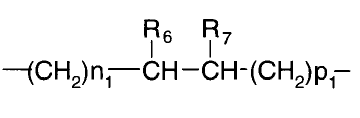 group,
where n1 and p1 are two integers the sum of which n1 + p1 is an integer chosen from 2, 3, 4 or 5;
R⁶ and R⁷ form together a covalent bond, or R⁶ and R⁷ form together with the carbon atoms to which they are connected the 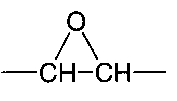 ring or a cycloalkyl comprising 3 to 6 members;
R⁴ represents an alkyl, aryl, or heteroaryl radical;
R⁵ represents a hydrogen, an alkyl or aralkyl radical;
or a pharmaceutically acceptable salt thereof.

2. Compound according to claim 1, in which X represents a linear or branched alkylene chain with 3 to 7 carbon atoms, this chain being able to contain one or more additional identical or different members chosen from -O-, -N(R⁵)-, -S-, -SO- or -SO₂- or a pharmaceutically acceptable salt thereof.

3. Compound according to claim 1 or 2, in which X represents an alkylene chain being able to contain a single member chosen from -O-, -N(R⁵)-, -S-, -SO- or -SO₂- or a pharmaceutically acceptable salt thereof.

4. Compound according to claim 1, in which X represents a 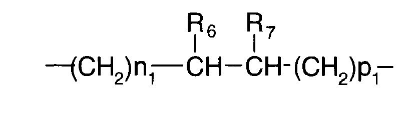 group,
where n1 and p1 are two integers the sum of which n1 + p1 is an integer chosen from 2, 3, 4 and 5;
R⁶ and R⁷ form together a covalent bond, or R⁶ and R⁷ form together with the carbon atoms to which they are connected the 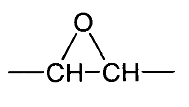 ring or a cycloalkyl comprising 3 to 4 members; or a pharmaceutically acceptable salt thereof.

5. Compound according to claim 4, in which n1 and p1 are equal.

6. Compound according to claim 1, in which X represents a (̵CH₂)n₂-X'-(CH₂)p₂ group, and X' represents an -O-, -N(R⁵)- or -S-, -SO-, -SO₂-, -(CH₂)- or 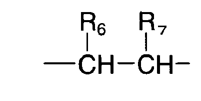 group, and n2 and p2 are integers the sum of which n2 + p2 is either an integer chosen from 3, 4, 5, 6 and 7 when X' represents an -O-, -N(R⁵)- or -S-, -SO-, -SO₂-group or an integer chosen from 2, 3, 4 and 5 when X' represents a 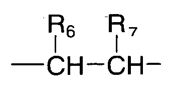 or - (CH₂)- group.

7. Compound according to claim 6, in which X' represents a 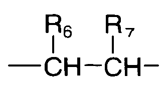 group.

8. Compound according to claim 6, in which X' represents an -O-, -N(R⁵)- or -(CH₂)- group.

9. Compound according to any one of claims 6 to 8, in which n2 and p2 are equal.

10. Compound according to one of claims 1 to 9, in which R³ represents an alkyl radical or the two R³ radicals form together with the carbon atom to which they are attached a cycloalkyl comprising 3 to 4 members.

11. Compound according to one of claims 1 to 10, in which R⁴ represents an alkyl radical.

12. Compound according to one of claims 1 to 11, in which R⁵ represents an alkyl radical.

13. Compound according to one of claims 1 to 12, in which R¹ is in para position.

14. Compound according to one of claims 1 to 13, in which R² is in meta position.

15. Compound according to one of claims 1 to 14, in which R⁶ and R⁷ form together a covalent bond.

16. Compound according to one of claims 1 to 14, in which R⁶ and R⁷ form together with the carbon atoms to which they are connected the 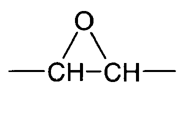 ring.

17. Compound according to one of claims 1 to 14, in which R⁶ and R⁷ form together with the carbon atoms to which they are connected a cycloalkyl comprising 3 to 6 members.

18. Compound according to one of claims 1 to 17, in which the alkyl radical represents a methyl group.

19. Compound of general formula **(I)** chosen from:
- 1,1'-butane-1,4-diylbis{5,5-dimethyl-3-[4-nitro-3-(trifluoromethyl)-phenyl]imidazolidine-2,4-dione}
- 1,1'-pentane-1,5-diylbis{5,5-dimethyl-3-[4-nitro-3-(trifluoromethyl)-phenyl]imidazolidine-2,4-dione}
- 1,1'-(oxydiethane-2,1-diyl)bis{5,5-dimethyl-3-[4-nitro-3-(trifluoromethyl)-phenyl]imidazolidine-2,4-dione}
- 4,4'-[pentane-1,5-diylbis(4,4-dimethyl-2,5-dioxoimidazolidine-3,1-diyl)]bis(2-methylbenzonitrile)
- 1,1'-(2Z)-but-2-ene-1,4-diylbis{5,5-dimethyl-3-[4-nitro-3-(trifluoromethyl) phenyl]imidazolidine-2,4-dione}
or a pharmaceutically acceptable salt thereof

20. Compound of general formula **(I),** consisting of the compound 1,1'-pentane-1,5-diylbis{5,5-dimethyl-3-[4-nitro-3-(trifluoromethyl)-phenyl]imidazolidine-2,4-dione} or of the compound 1,1'-(2Z)-but-2-ene-1,4-diylbis{5,5-dimethyl-3-[4-nitro-3-(trifluoromethyl) phenyl]imidazolidine-2,4-dione or a pharmaceutically acceptable salt thereof.

21. Process for the preparation of a compound of formula **(I)** as defined in claim 1, comprising a stage which consists of
(i) condensing two equivalents of the arylhydantoins of general formula **(II)** 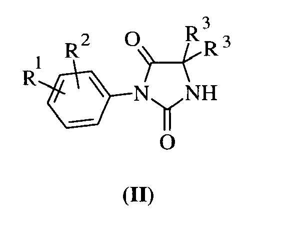 in which R² and R³ are as defined in claim 1 and R¹ is a nitro or cyano group, with a derivative of general formula Gp¹-X-Gp², Gp¹ and Gp² being leaving groups, and X being as defined previously, in the presence of a strong base, in order to form the compound of general formula **(I)** 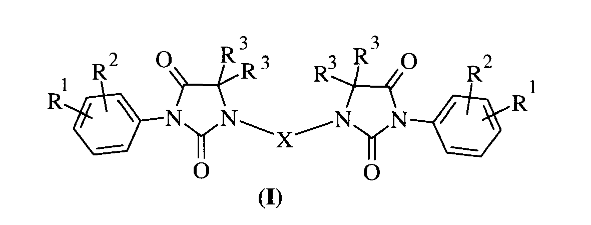 in which R², R³ and X are as defined in claim 1 and R¹ is a nitro or cyano group;
optionally, if R¹ is a nitro group, the process can moreover comprise a stage of:
(ii) reducing the nitro group in order to obtain a compound of formula (III) 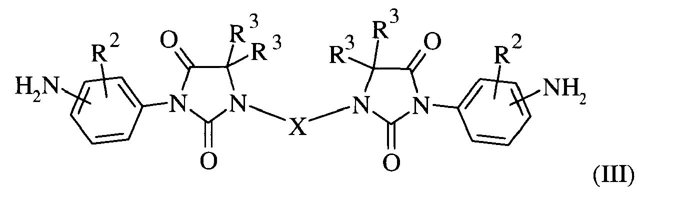 optionally, the process can moreover comprise a stage chosen from:
(iii) reacting a compound of formula (III) obtained in stage (ii) with an acid chloride of general formula R⁴-COCl, in which R⁴ is as defined in claim 1, in order to obtain a compound of formula (IV) 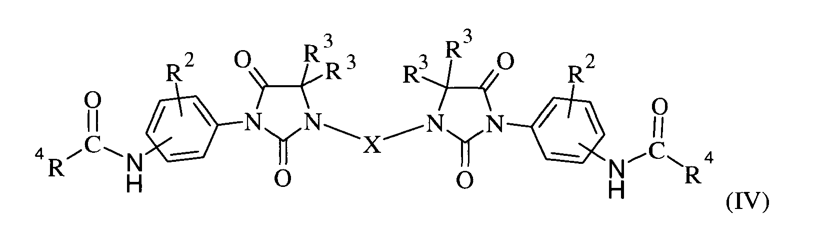
(iv) reacting a compound of formula (III) obtained in stage (ii) with a chloroformate of general formula R⁴-O-CO-Cl in which R⁴ is as defined in claim 1 in order to obtain a compound of formula (V) 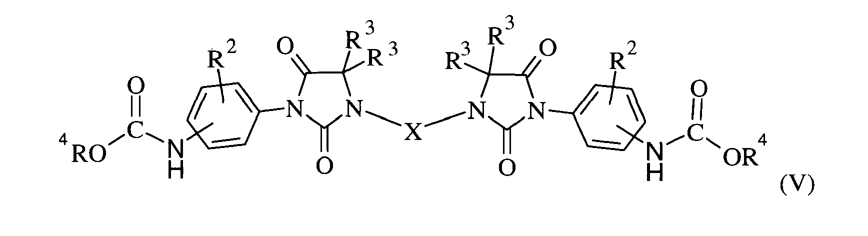 optionally, if R⁶ and R⁷ form together a covalent bond, the process can moreover comprise a stage of:
(v) oxidizing the compound of formula (I) in which R⁶ and R⁷ form together a covalent bond, the double bond thus formed by R⁶ and R⁷ in order to obtain a compound of formula (VI)

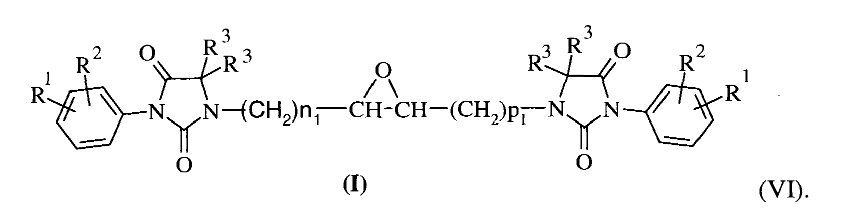

22. As a medicament, a compound according to one of claims 1 to 20.

23. Pharmaceutical compositions containing, as active ingredient, at least one compound of formula **(I)** as defined in one of claims 1 to 20, in combination with a pharmaceutically acceptable support.

24. Use of a compound of formula **(I)** according to one of claims 1 to 20, for the preparation of a medicament intended to treat cancers.

25. Use according to claim 24, in which the medicament is intended to treat a hormone-dependent cancer.

26. Use according to claim 24 in which the medicament is intended to treat a cancer expressing androgen receptors.

27. Use according to one of claims 24 to 26 in which the medicament is intended to treat a cancer of the breast or prostate.
